# EUROPEAN PATENT APPLICATION

(11) **EP 4 311 831 A1**
(43) Date of publication of application: **31.01.2024**
(21) Application number: 22187550.3
(22) Date of filing: 28.07.2022
(51) Int. Cl.: C07H 9/04, C09K 23/00, C11D 1/66

(54) **BIOBASED SURFACTANT**

(71) Applicant: ECOLE POLYTECHNIQUE FEDERALE DE LAUSANNE (EPFL), 1015 Lausanne (CH)
(72) Inventor: Bertella, Stefania, 1018 Lausanne (CH); Komarova, Anastasiia, 1022 Chavannes près Renens (CH); Sun, Songlan, 1024 Ecublens (CH); Luterbacher, Jeremy, 1022 Chavannes près Renens (CH)
(74) Representative: Schaad, Balass, Menzl & Partner AG

(57) **Abstract**

The present invention relates to a compound of the general formula (I), (II) and (III) 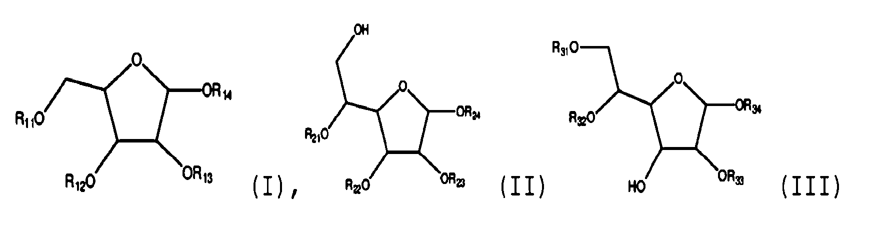 , more specifically of formula (Ia), (Ib), (Ic) 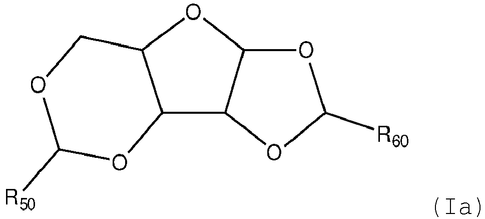 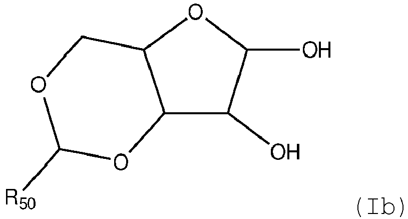 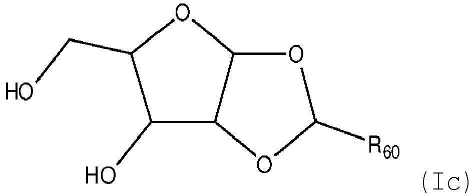 wherein R₁₁ and R₁₂ or R₂₁ and R₂₂ or R₃₁ and R₃₂ are both hydrogen or form together with CHR₅₀ a cyclic moiety or one of R₁₁ and R₁₂ or R₂₁ and R₂₂ or R₃₁ and R₃₂ is hydrogen and the other is - CH₂R₇₀ and/or wherein R₁₃ and R₁₄ or R₂₃ and R₂₄ or R₃₃ and R₃₄ are both hydrogen or form together with CHR₆₀ a cyclic moiety, R₅₀ and R₆₀ are different form each other and are selected from the group consisting of -R₇₀, -ZR₇₀, -Z-OH, -Z-NH₂, -Z-SH, -Z-OC(O)R₇₀, -OC(O)R₇₀, -COOH, -C(O)NH₂, -C(O)NH-R₇₀, -C(O)N-(R₇₀)₂, -COOR₇₀, -Z-COOH, -Z-C(O)NH-R₇₀, -Z-C(O)NH₂, -Z-C(O)N-(R₇₀)₂, - Z-COOR₇₀, -CH(COOH)₂, -CH(COOR₇₀)₂, and -Z-SO₃⁻, wherein R₇₀ is a linear or branched C₁ to C₂₀ alkyl, (C₁ to C₁₀)-alkyloxy-(C₁ to C₁₀)-alkyl, C₂ to C₁₀ alkenyl, C₆ to C₁₂ aryl, C₃ to C₁₀ cycloalkyl, cycloalkylalkyl and cycloalkylalkenyl, wherein Z is a linear or branched C₁ to C₁₀ alkyl, linear or branched C₃ to C₁₀ cycloalkyl, cycloalkylalkyl and cycloalkylalkenyl, a linear or branched C₆ to C₁₀ aryl or a (C₁ to C₁₀)-alkyloxy-(C₁ to C₁₀)-alkyl, with the proviso that not all of R₁₁, R₁₂, R₁₃ and R₁₄ or R₂₁, R₂₂, R₂₃ and R₂₄ or R₃₁, R₃₂, R₃₃ and R₃₄ are hydrogen and if one of R₁₁ and R₁₂ or R₂₁ and R₂₂ or R₃₁ and R₃₂ is hydrogen and the other is -CH₂R₇₀, R₁₃ and R₁₄ or R₂₃ and R₂₄ or R₃₃ and R₃₄ form together with -CHR₆₀ a cyclic moiety, wherein R₆₀ is R₇₀.
Those compounds are useful as as biobased surfactants.

## Description

The present invention relates to new compounds and their use as biobased surfactants.

Surfactants are a class of chemicals used in a wide range of applications and fields such as the detergent, medical, pharmaceutical, food and paint industries. With the advent of the COVID-19 global pandemic, the demand of surfactants has risen, since these compounds contained in soaps and sanitizing compositions have the ability of disrupting the lipidic membrane of the SARS-CoV-2 virus making said virus inefficient.

Given the huge market and big demand for surfactants, it is important that their production does not rely only on fossil-based sources, but more and more it is fundamental that they are synthesized and sourced from renewable feedstocks that don't impact as much on the second current global challenge, climate change.

Moreover, as the lifetime of surfactant is short, given that they are generally washed away and tend to end up in the open, their structure had degradability products have to be compatible with the environment they end up in, so that they neither accumulate nor cause harm to the diverse species and ecosystems.

Bio-based surfactants (Sophorolipids, Rhamnolipids) are commercially available. Sophorolipids are commercialised by Ecover^{™}, Saraya^{™}, Intobio^{™}, Evonik^{™} and Allied Carbon Solutions^{™}. All of them have a critical micelle concentration CMC 7-10-fold less than CMC of SDS but need to be produced with yeasts (average fermentations times: 7 days) and with fatty acids of tropical plant origin, that still pose an environmental pressure, due to deforestation issues.

US2021353517A1 discloses a process for producing a bio-based surfactant comprising an alkyl disulphate salt comprises the steps of methanolysis of medium chain length polyhydroxyalkanoic acid (mcl-PHA) to provide hydroxy fatty acid methyl ester monomers (HFAME's), reduction of the HFAME's to provide 1,3 alkyl diols, sulphation of the 1,3 alkyl diols to provide 1,3 alkyl disulphates, and neutralisation of the alkyl disulphates to provide a bio-based surfactant comprising 1,3 alkyl disulphate salt.

Particularly, carbohydrates (or their derivatives)-based surfactants have received a lot of attention and development for the variety of possible chemical reactions that are possible on the hydroxyl group of their core structure. Typical carbohydrate-based molecules or derivatives that can be chemically reacted to form surfactants are xylose, glucose, sorbitol, sorbitan, arabinose, isosorbide, and uronic acid. Amongst the possible reaction that have been studied in making sugar-based surfactants the most common are the esterification of carbohydrate hydroxyl group with long chain acids, such as the case of the commercial Span and Tween derived from sorbitol. Other important reactions of carbohydrates to produce surfactants are etherification to form simple ether or reactions with long-chain aldehydes to form stable acetals. Finally, in the case of uronic acid, amide-based surfactants can be synthesized. Some of these reactions, although successful (such as the case of commercial esters based on sorbitan), are based on sugar or derivatives that undergo several synthetic steps of reduction, isomerization or dehydration of more abundant sugars before becoming surfactants, increasing their environmental and energetical impact with each synthetic step.

The problem of the present invention is therefore to provide bio-based surfactant which can be synthesized in a few steps directly from renewable sources.

The problem is solved by the compounds according to claim 1. Further preferred embodiments are subject of the dependent claims.

As explained in detail below, compounds of formula I, II and III can be obtained based on aldehyde assisted biomass fractionation and acetal Functionalization from carbohydrate-based molecules. The compounds of the present invention are biodegradable and have no negative impact on human and animal health. In addition, they have no or only a very limited negative influence on fauna, flora, and ecosystems since they are derived from renewable resources. Furthermore, the synthesis of the compounds according to the present invention is simple which allows a large-scale bio-based surfactant production.

The present invention relates to a compound of the general formula (I), (II) and (III) 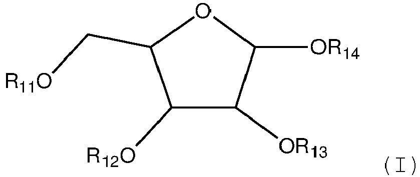 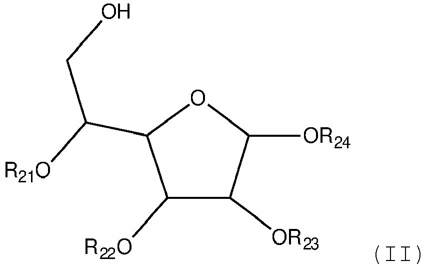 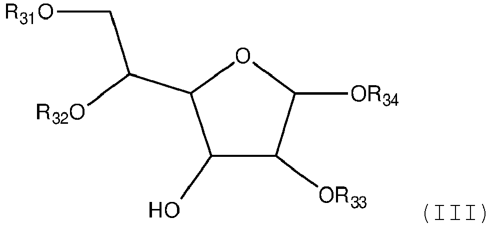
wherein R₁₁ and R₁₂ or R₂₁ and R₂₂ or R₃₁ and R₃₂ are both hydrogen or form together with CHR₅₀ a cyclic moiety or one of R₁₁ and R₁₂ or R₂₁ and R₂₂ or R₃₁ and R₃₂ is hydrogen, and the other is - CH₂R₇₀ and
wherein R₁₃ and R₁₄ or R₂₃ and R₂₄ or R₃₃ and R₃₄ are both hydrogen or form together with CHR₆₀ a cyclic moiety,
R₅₀ and R₆₀ are different from each other and are selected from the group consisting of -R₇₀, -ZR₇₀, -Z-OH, -Z-NH₂, -Z-SH, -Z-OC(O)R₇₀, -OC(O)R₇₀, -COOH, -C(O)NH₂, -C(O)NH-R₇₀, -C(O)N-(R₇₀)₂, -COOR₇₀, -Z-COOH, -Z-C(O)NH-R₇₀, -Z-C(O)NH₂, -Z-C(O)N-(R₇₀)₂, - Z-COOR₇₀, -CH(COOH)₂, -CH(COOR₇₀)₂, and -Z-SO₃⁻
wherein R₇₀ is selected from the group consisting of a linear or branched C₁ to C₂₀ alkyl, (C₁ to C₁₀) -alkyloxy- (C₁ to C₁₀)-alkyl, C₂ to C₁₀ alkenyl, C₆ to C₁₂ aryl, C₃ to C₁₀ cycloalkyl, cycloalkylalkyl and cycloalkylalkenyl,
wherein Z is selected from the group consisting of a linear or branched C₁ to C₁₀ alkyl, linear or branched C₃ to C₁₀ cycloalkyl, a linear or branched C₆ to C₁₀ aryl, a (C₁ to C₁₀)-alkyloxy-(C₁ to C₁₀)-alkyl, cycloalkylalkyl and cycloalkylalkenyl,
with the proviso that
not all of R₁₁, R₁₂, R₁₃ and R₁₄ or R₂₁, R₂₂, R₂₃ and R₂₄ or R₃₁, R₃₂, R₃₃ and R₃₄ are hydrogen and
if one of R₁₁ and R₁₂ or R₂₁ and R₂₂ or R₃₁ and R₃₂ is hydrogen and the other is -CH₂R₇₀, R₁₃ and R₁₄ or R₂₃ and R₂₄ or R₃₃ and R₃₄ form together with -CHR₆₀ a cyclic moiety, wherein R₆₀ is R₇₀.

The term "linear or branched C₁ to C₂₀ alkyl" as a residue refers to a linear or branched hydrocarbon chain containing 1 to 20 of carbon atoms. Examples of said term as used herein include methyl, ethyl, n-propyl, isopropyl, n-butyl, isobutyl, sec-butyl, tert-butyl, n-pentyl, 3-methylbutyl, sec-pentyl, and 2-methylbutan-2-yl.

The term "a (C₁ to C₁₀)-alkyloxy- (C₁ to C₁₀)-alkyl" as a residue refers to a residue -(C₁-C₁₀)-O-(C₁-C₁₀)-, i.e., a linear or branched hydrocarbon chain containing preferably 1 to 20 of carbon atoms, of which at least two are singularly bonded to oxygen. Examples of said term as used herein include 2-ethylethoxy, 2-ethylpropoxy, 2-ethylpropoxy, 1-ethylbutoxy, 3-ethylbutoxy, and 2-ethylpropoxy.

The term "linear or branched C₂ to C₂₀ alkenyl" as a residue refers to a linear or branched hydrocarbon chain containing 2 to 20 of carbon atoms which contains at least one double bond.

The term "C₆ to C₁₂ aryl" refers to an aromatic carbon ring system having any suitable number of ring atoms and any suitable number of rings. Aryl groups can include any suitable number of carbon ring atoms, such as, 6, 7, 8, 9, 10, 11 or 12 ring atoms, as well as from 6 to 10 or 6 to 12 ring members. Aryl groups can be monocyclic, fused to form bicyclic or tricyclic groups, or linked by a bond to form a biaryl group. Representative aryl groups include phenyl, naphthyl and biphenyl. Some aryl groups have from 6 to 12 ring members, such as phenyl, naphthyl or biphenyl. Other aryl groups have from 6 to 10 ring members, such as phenyl or naphthyl. Some other aryl groups have 6 ring members, such as phenyl.

The term "cycloalkyl" means a non-aromatic monocyclic ring system comprising 3 to 10 carbon atoms, preferably 5 to 10 carbon atoms. Preferred cycloalkyl rings contain about 5 to about 7 ring atoms. Non-limiting examples of suitable monocyclic cycloalkyls include cyclopentyl, cyclohexyl, cycloheptyl and the like.

The term "cycloalkylalkyl" means a residue -RₐR_{b} where Rₐ is an alkylene group having 1 to 8 carbon atoms and R_{b} is cycloalkyl group as defined above.

The term "cycloalkylalkenyl" means a residue -Rₐ'R_{b} where Rₐ' is an alkenylene group having 2 to 8 carbon atoms and R_{b} is cycloalkyl group as defined above.

The compound of the present invention is preferably used as a non-ionic surfactant, which in general comprises an acetal-stabilized sugar core and residual groups R (for example R₁₁, or R₃₁) and has the same properties as polyoxyethylene-based surfactants and sugar-derived polyols.

Polyoxyethylene-based surfactants exhibit inverse solubility characteristics and may precipitate with temperature increase, because of the broken hydrogen at under high temperatures.

Sugar-derived polyols such as compounds having a sugar core functionalized with pH-responsive functional group, e.g. a carboxylic acid (-COOH), or an amine (-NH₂), are very pH-responsive. Their solubility strongly depends on the pH and some of the linkages are not very stable either under very acidic or under very basic condition Therefore, they can be easily degraded after use without burdening the environment. On the other hand, with the exchange of the functional group a sugar-derived polyol can be adapted from working in a lower pH range to work in the adapted form in a higher pH range.

These properties allow for a complex, multi responsive behaviour of these compounds, when temperature and pH of the solution are changed. Resulting potential applications could comprise the use as a thermo-responsive and chelating aminoacid modified surfactants, temperature-responsive functional surfactants, tethered lipid membranes with a variable hydrophilic cushion or hydrophilic and temperature-responsive drug carriers. Further these compounds can be used as surfactants in household and industrial cleaning agents.

Due to the variety of residual groups of the claimed compound, different kinds of effects can be expected.

Compounds containing ester groups are more sensitive towards hydrolyses, especially in alkaline conditions. This means that esters are preferably used as emulsifiers and stabilizers under neutral conditions in personal care, food and pharmaceutical applications.

Compounds containing amide groups are relatively more stable than compounds containing an ester group and tend to be hydrolysed only in harsh conditions such as strong acid (e.g. H₂SO₄) in combination with heat or strong base (e.g. NaOH) in combination with heat.

Compounds containing alkyl ethers groups are relatively stable in alkaline surroundings but under acidic catalysis they can turn into furans, and subsequently humins (sugar degradation products). They can be used in cleaning under mild conditions, such as in personal care, dish-washing liquids, etc., but also for applications where alkaline conditions are required. It is expected that compounds with alkyl ethers groups are resistant to hard water conditions (between 120 - 180 mg CaCO₃/L).

Compounds containing carboxylic acid groups opens up a multitude of possible applications due to their pH-responsive character. The degree of ionization of carboxylic acid residue depends on the pH of the solution. Moreover, the high reactivity of the carboxylic acid residues enables the preparation of surfactants tailored to specific purposes. Due to the simultaneous presence of the ether units and the carboxylic acid residues, clear solutions of surfactants with long alkyl chains (C₁₂ - C₁₈)
can be obtained in alkaline, as well as acidic solutions which are remarkably stable in hard water and high salinity conditions. In combination with their low toxicity, stability to hydrolysis, oxidizing agents, high temperatures, they will find application in different fields, most notably as detergents and in home and body-care products, for enhanced oil recovery and as additives in the textile and metal processing industry.

In a preferred embodiment of the present invention compound of formula (I), (II) or (III) is selected from the group consisting of 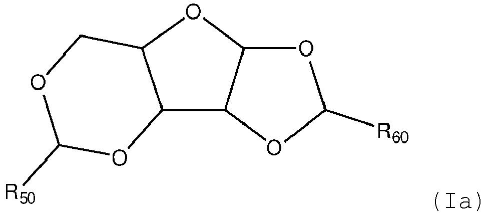 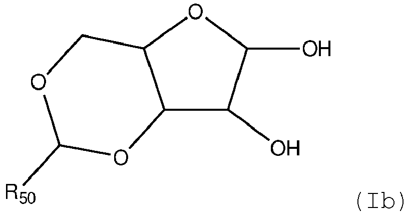 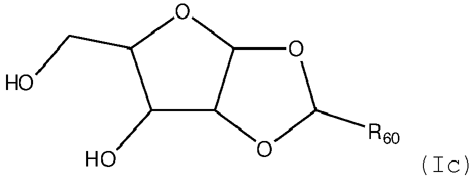 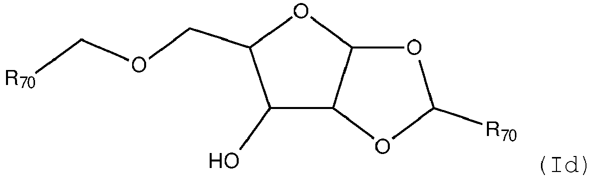 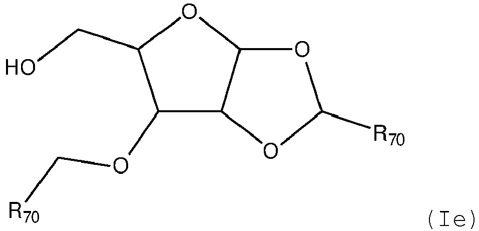 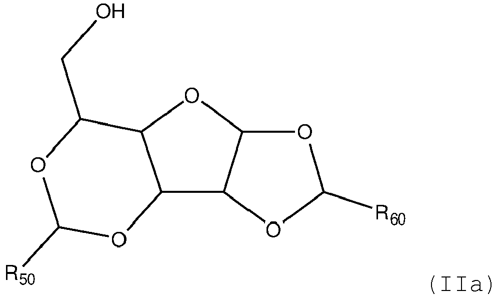 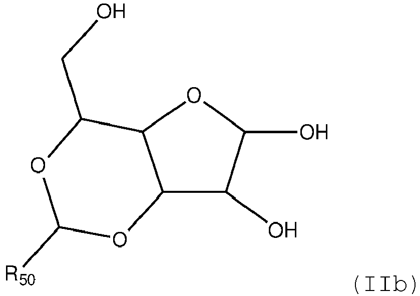 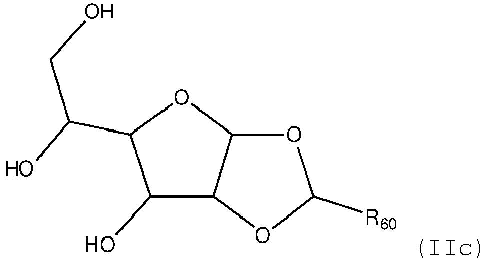 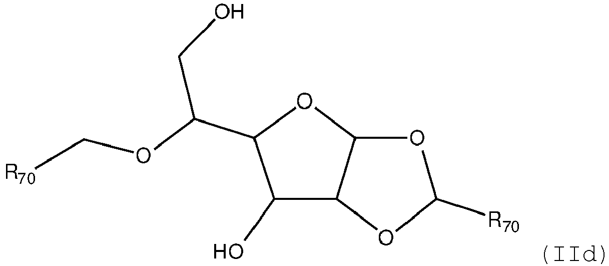 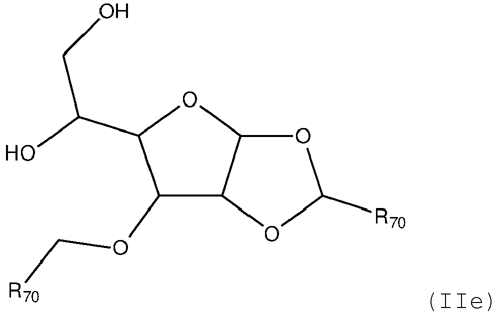 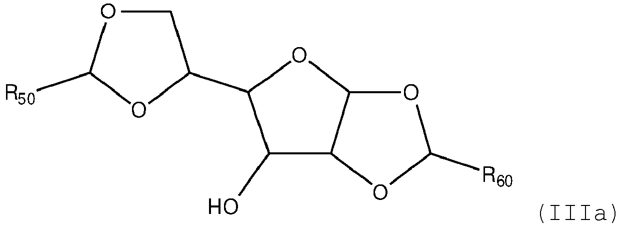 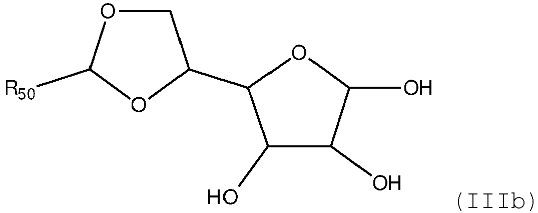 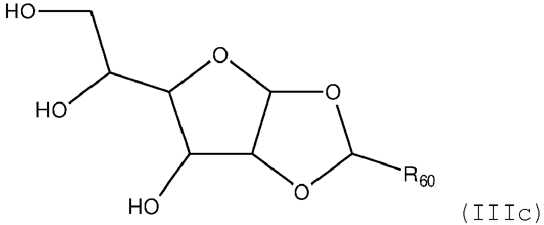 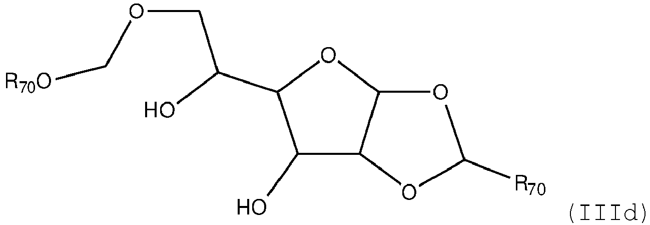 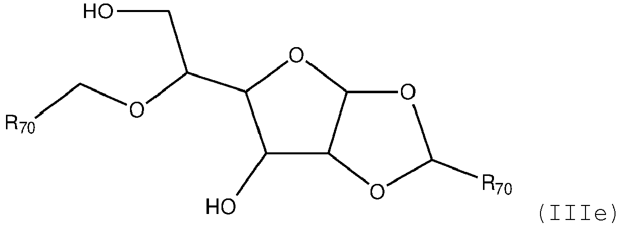 wherein R₅₀, R₆₀ and R₇₀ have the same definition as above.

In compound of formula (I), (II) or (III) preferably at least one of R₅₀ and R₆₀ is selected from the group consisting of -Z-OC(O)R₇₀, -COOR₇₀ and -Z-COOR₇₀ and the other is selected from the group consisting of -Z-OH, -COOH and -Z-COOH.

One embodiment of the present invention relates to compound Ia 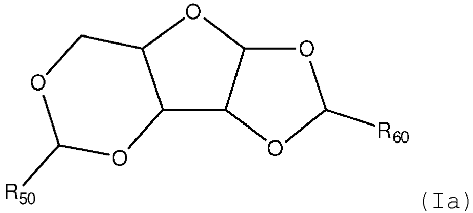 wherein R₅₀ and R₆₀ are as defined below:

| R₅₀ | R₆₀ | R₅₀ | R₆₀ |
|---|---|---|---|
| -Z-OC(O)-R₇₀ | -Z-OH | -C(O)NH-R₇₀ | -Z-OH |
| -C(O)-OR₇₀ | -Z-OH | -Z-C(O)NH-R₇₀ | -Z-OH |
| -Z-C(O)O-R₇₀ | -Z-OH | -CH (COOR₇₀)₂ | -Z-OH |
| -Z-OC(O)-R₇₀ | -COOH | -C(O)NH-R₇₀ | -COOH |
| -C(O)-OR₇₀ | -COOH | -Z-C(O)NH-R₇₀ | -COOH |
| -Z-C(O)O-R₇₀ | -COOH | -CH (COOR₇₀)₂ | -COOH |
| -Z-OC(O)-R₇₀ | -Z-COOH | -C(O)NH-R₇₀ | -Z-COOH |
| -C(O)-OR₇₀ | -Z-COOH | -Z-C(O)NH-R₇₀ | -Z-COOH |
| -Z-C(O)O-R₇₀ | -Z-COOH | -CH (COOR₇₀)₂ | -Z-COOH |
| -Z-OC(O)-R₇₀ | -CH-(COOH)₂ | -C(O)NH-R₇₀ | -CH-(COOH)₂ |
| -C(O)-OR₇₀ | -CH-(COOH)₂ | -Z-C(O)NH-R₇₀ | -CH-(COOH)₂ |
| -Z-C(O)O-R₇₀ | -CH-(COOH)₂ | -CH (COOR₇₀)₂ | -CH-(COOH)₂ |
| -Z-OH | -Z-OC(O)-R₇₀ | -Z-OH | -C(O)NH-R₇₀ |
| -Z-OH | -C(O)-OR₇₀ | -Z-OH | -Z-C(O)NH-R₇₀ |
| -Z-OH | -Z-C(O)O-R₇₀ | -Z-OH | -CH (COOR₇₀)₂ |
| -COOH | -Z-OC(O)-R₇₀ | -COOH | -C(O)NH-R₇₀ |
| -COOH | -C(O)-OR₇₀ | -COOH | -Z-C(O)NH-R₇₀ |
| -COOH | -Z-C(O)O-R₇₀ | -COOH | -CH (COOR₇₀)₂ |
| -Z-COOH | -Z-OC(O)-R₇₀ | -Z-COOH | -C(O)NH-R₇₀ |
| -Z-COOH | -C(O)-OR₇₀ | -Z-COOH | -Z-C(O)NH-R₇₀ |
| -Z-COOH | -Z-C(O)O-R₇₀ | -Z-COOH | -CH (COOR₇₀)₂ |
| -CH-(COOH)₂ | -Z-OC(O)-R₇₀ | -CH-(COOH)₂ | -C(O)NH-R₇₀ |
| -CH-(COOH)₂ | -C(O)-OR₇₀ | -CH-(COOH)₂ | -Z-C(O)NH-R₇₀ |
| -CH-(COOH)₂ | -Z-C(O)O-R₇₀ | -CH-(COOH)₂ | -CH (COOR₇₀)₂ |

One embodiment of the present invention relates to compound Ia 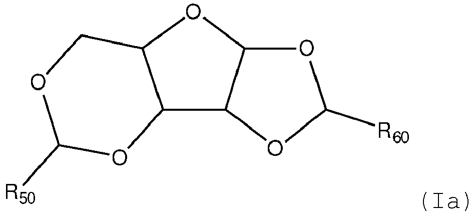 wherein R₅₀ and R₆₀ are as defined below:

| R₅₀ | R₆₀ |
|---|---|
| - (CH₂)₆CH₃ | - (CH₂)₆CH₃ |
| - (CH₂)₈CH₃ | - (CH₂)₈CH₃ |
| - (CH₂)₁₀CH₃ | - (CH₂)₁₀CH₃ |

One embodiment of the present invention relates to compound Ib 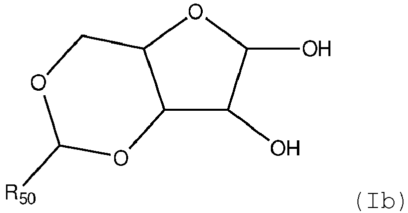 wherein R₅₀ is selected from the group consisting of -R₇₀, -Z-OC(O)-R₇₀, -C(O)-OR₇₀, -Z-C(O)O-R₇₀, -C(O)NH-R₇₀, -Z-C(O)NH-R₇₀, -CH(COOR₇₀)₂, preferably selected from the group consisting of -Z-C(O)-OR₇₀, -C(O)-OR₇₀ and -R₇₀, and most preferably selected from the group consisting of -C(O)-O(CH₂)₄CH₃, -C(O)-O(CH₂)₅CH₃, -C(O)-O(CH₂)₆CH₃, -C(O)-O(CH₂)₇CH₃, -C (O)-O(CH₂)₈CH₃, -C(O)-O(CH₂)₉CH₃, -C (O)-O(CH₂)₁₀CH₃, -C(O)-O(CH₂)₁₁CH₃, -C(O) - O(CH₂)₁₂CH₃, -C(O)-O(CH₂)₁₃CH₃, -C(O)-O(CH₂)₁₄CH₃, -CH₂-C(O)-O(CH₂)₄CH₃, -CH₂-C(O)-O(CH₂)₅CH₃, -CH₂-C(O)-O(CH₂)₆CH₃, -CH₂-C (O)-O(CH₂)₇CH₃, -CH₂-C(O)-O(CH₂)₈CH₃, -CH₂-C(O)-O(CH₂)₉CH₃, - CH₂-C (O)-O(CH₂)₁₀CH₃, -CH₂-C(O)-O(CH₂)₁₁CH₃, -CH₂-C(O)-O(CH₂)₁₂CH₃, -CH₂-C(O)-O(CH₂)₁₃CH₃, -CH₂-C(O)-O(CH₂)₁₄CH₃, - (CH₂)₂-C (O)-O(CH₂)₄CH₃, - (CH₂)₂-C (O)-O(CH₂)₅CH₃, - (CH₂)₂-C(O) - O(CH₂)₆CH₃, - (CH₂)₂-C(O)-O(CH₂)₇CH₃, - (CH₂)₂-C(O)-O(CH₂)₈CH₃, - (CH₂)₂-C(O)-O(CH₂)₉CH₃, - (CH₂)₂-C(O)-O(CH₂)₁₀CH₃, - (CH₂)₂-C(O) - O(CH₂)₁₁CH₃, - (CH₂)₂-C(O)-O(CH₂)₁₂CH₃, - (CH₂)₂-C(O)-O(CH₂)₁₃CH₃, - (CH₂)₂-C(O)-O(CH₂)₁₄CH₃, - (CH₂)₃-C(O)-O(CH₂)₄CH₃, - (CH₂)₃-C(O)-O(CH₂)₅CH₃, - (CH₂)₃-C(O)-O(CH₂)₆CH₃, - (CH₂)₃-C(O)-O(CH₂)₇CH₃, - (CH₂)₃-C(O)-O(CH₂)₈CH₃, - (CH₂)₃-C(O)-O(CH₂)₉CH₃, - (CH₂)₃-C(O)-O(CH₂)₁₀CH₃, - (CH₂)₃-C(O)-O(CH₂)₁₁CH₃, - (CH₂)₃-C(O)-O(CH₂)₁₂CH₃, - (CH₂)₃-C(O)-O(CH₂)₁₃CH₃, - (CH₂)₃-C(O)-O(CH₂)₁₄CH₃, - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, and - (CH₂)₁₄CH₃.

One embodiment of the present invention relates to compound Ic 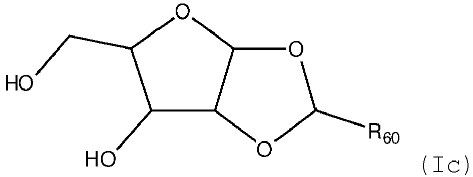 wherein R₆₀ is selected from the group consisting of -R₇₀, -Z-OC(O)-R₇₀, -C(O)-OR₇₀, -Z-C (O)O-R₇₀, -C(O)NH-R₇₀, -Z-C(O)NH-R₇₀, -CH(COOR₇₀)₂, preferably selected from the group consisting of -Z-C(O)-OR₇₀, -C(O)-OR₇₀ and -R₇₀, and most preferably selected from the group consisting of -C(O)-O(CH₂)₄CH₃, -C(O)-O(CH₂)₅CH₃, -C(O)-O(CH₂)₆CH₃, -C(O)-O(CH₂)₇CH₃, -C(O)-O(CH₂)₈CH₃, -C(O)-O(CH₂)₉CH₃, -C(O)-O(CH₂)₁₀CH₃, -C(O)-O(CH₂)₁₁CH₃, -C(O)-O(CH₂)₁₂CH₃, -C(O)-O(CH₂)₁₃CH₃, -C(O)-O(CH₂)₁₄CH₃, -CH₂-C(O)-O(CH₂)₄CH₃, -CH₂-C(O)-O(CH₂)₅CH₃, -CH₂-C(O)-O(CH₂)₆CH₃, -CH₂-C(O)-O(CH₂)₇CH₃, -CH₂-C(O)-O(CH₂)₈CH₃, -CH₂-C(O)-O(CH₂)₉CH₃, - CH₂-C(O)-O(CH₂)₁₀CH₃, -CH₂-C(O)-O(CH₂)₁₁CH₃, -CH₂-C(O)-O(CH₂)₁₂CH₃, -CH₂-C(O)-O(CH₂)₁₃CH₃, -CH₂-C(O)-O(CH₂)₁₄CH₃, - (CH₂)₂-C(O)-O(CH₂)₄CH₃, - (CH₂)₂-C(O)-O(CH₂)₅CH₃, - (CH₂)₂-C (O) - O(CH₂)₆CH₃, - (CH₂)₂-C(O)-O(CH₂)₇CH₃, - (CH₂)₂-C(O)-O(CH₂)₈CH₃, - (CH₂)₂-C(O)-O(CH₂)₉CH₃, - (CH₂)₂-C(O)-O(CH₂)₁₀CH₃, - (CH₂)₂-C(O)-O(CH₂)₁₁CH₃, - (CH₂)₂-C(O)-O(CH₂)₁₂CH₃, - (CH₂)₂-C(O)-O(CH₂)₁₃CH₃, - (CH₂)₂-C(O)-O(CH₂)₁₄CH₃, - (CH₂)₃-C(O)-O(CH₂)₄CH₃, - (CH₂)₃-C(O)-O(CH₂)₅CH₃, - (CH₂)₃-C(O)-O(CH₂)₆CH₃, - (CH₂)₃-C(O)-O(CH₂)₇CH₃, - (CH₂)₃-C(O)-O(CH₂)₈CH₃, - (CH₂)₃-C(O)-O(CH₂)₉CH₃, - (CH₂)₃-C(O)-O(CH₂)₁₀CH₃, - (CH₂)₃-C(O)-O(CH₂)₁₁CH₃, - (CH₂)₃-C(O)-O(CH₂)₁₂CH₃, - (CH₂)₃-C(O)-O(CH₂)₁₃CH₃, - (CH₂)₃-C(O)-O(CH₂)₁₄CH₃, - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, and - (CH₂)₁₄CH₃.

One embodiment of the present invention relates to compound Ic 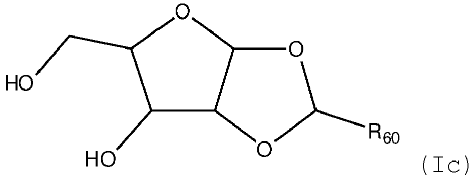 wherein R₆₀ is -R₇₀ and -R₇₀ is selected from the group consisting of -CH₃, -(CH₂)CH₃, -(CH₂)₂CH₃, -(CH₂)₃CH₃, -(CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, - (CH₂)₁₄CH₃, - (CH₂)₁₅CH₃, - (CH₂)₁₆CH₃, - (CH₂)₁₇CH₃, - (CH₂)₁₈CH₃ and - (CH₂)₁₉CH₃, preferably -R₇₀ is selected from the group consisting of - (CH₂)₆CH₃, - (CH₂)₇CH₃, - (CH₂)₈CH₃, - (CH₂)₉CH₃ and - (CH₂)₁₀CH₃, more preferably -R₇₀ is -(CH₂)₁₀CH₃.

One embodiment of the present invention relates to compound Id 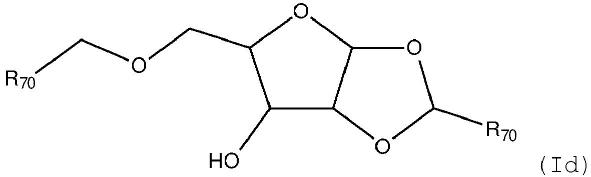 wherein R₇₀ is selected from the group consisting of -CH₃, - (CH₂)CH₃, - (CH₂)₂CH₃, - (CH₂)₃CH₃, - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, - (CH₂)₁₄CH₃, - (CH₂)₁₅CH₃, - (CH₂)₁₆CH₃, - (CH₂)₁₇CH₃, - (CH₂)₁₈CH₃ and - (CH₂)₁₉CH₃,
preferably R₇₀ is selected from the group consisting of - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, - (CH₂)₁₄CH₃,
more preferably R₇₀ is selected from the group consisting of - (CH₂)₆CH₃, - (CH₂)₇CH₃, - (CH₂)₈CH₃, - (CH₂)₉CH₃ and - (CH₂)₁₀CH₃ most preferably R₇₀ is -(CH₂)₁₀CH₃.

One embodiment of the present invention relates to compound Ie 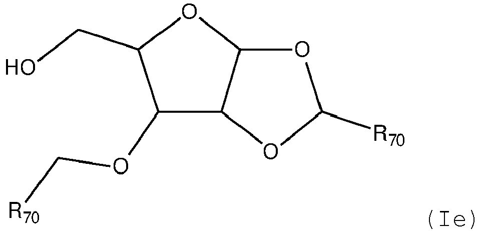
wherein R₇₀ is selected from the group consisting of -CH₃, - (CH₂)CH₃, -(CH₂)₂CH₃, -(CH₂)₃CH₃, -(CH₂)₄CH₃, -(CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, - (CH₂)₁₄CH₃, - (CH₂)₁₅CH₃, - (CH₂)₁₆CH₃, - (CH₂)₁₇CH₃, - (CH₂)₁₈CH₃ and - (CH₂)₁₉CH₃,
preferably R₇₀ is selected from the group consisting of - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃ and - (CH₂)₁₄CH₃,
more preferably R₇₀ is selected from the group consisting of - (CH₂)₆CH₃, - (CH₂)₇CH₃, - (CH₂)₈CH₃, - (CH₂)₉CH₃ and - (CH₂)₁₀CH₃ most preferably R₇₀ is -(CH₂)₁₀CH₃.

One embodiment of the present invention relates to compound IIa 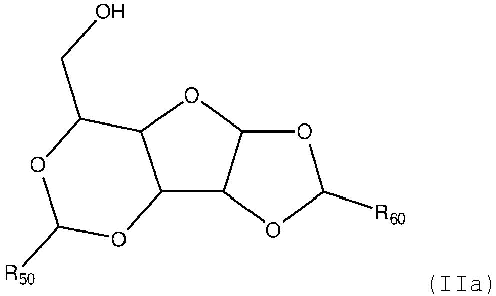 wherein R₅₀ and R₆₀ are as defined below:

| R₅₀ | R₆₀ | R₅₀ | R₆₀ |
|---|---|---|---|
| -Z-OC(O)-R₇₀ | -Z-OH | -C(O)NH-R₇₀ | -Z-OH |
| -C(O)-OR₇₀ | -Z-OH | -Z-C(O)NH-R₇₀ | -Z-OH |
| -Z-C(O)O-R₇₀ | -Z-OH | -CH (COOR₇₀)₂ | -Z-OH |
| -Z-OC(O)-R₇₀ | -COOH | -C(O)NH-R₇₀ | -COOH |
| -C(O)-OR₇₀ | -COOH | -Z-C(O)NH-R₇₀ | -COOH |
| -Z-C(O)O-R₇₀ | -COOH | -CH (COOR₇₀)₂ | -COOH |
| -Z-OC(O)-R₇₀ | -Z-COOH | -C(O)NH-R₇₀ | -Z-COOH |
| -C(O)-OR₇₀ | -Z-COOH | -Z-C(O)NH-R₇₀ | -Z-COOH |
| -Z-C(O)O-R₇₀ | -Z-COOH | -CH (COOR₇₀)₂ | -Z-COOH |
| -Z-OC(O)-R₇₀ | -CH-(COOH)₂ | -C(O)NH-R₇₀ | -CH-(COOH)₂ |
| -C(O)-OR₇₀ | -CH-(COOH)₂ | -Z-C(O)NH-R₇₀ | -CH-(COOH)₂ |
| -Z-C(O)O-R₇₀ | -CH-(COOH)₂ | -CH (COOR₇₀)₂ | -CH-(COOH)₂ |
| -Z-OH | -Z-OC(O)-R₇₀ | -Z-OH | -C(O)NH-R₇₀ |
| -Z-OH | -C(O)-OR₇₀ | -Z-OH | -Z-C(O)NH-R₇₀ |
| -Z-OH | -Z-C(O)O-R₇₀ | -Z-OH | -CH(COOR₇₀)₂ |
| -COOH | -Z-OC(O)-R₇₀ | -COOH | -C(O)NH-R₇₀ |
| -COOH | -C(O)-OR₇₀ | -COOH | -Z-C(O)NH-R₇₀ |
| -COOH | -Z-C(O)O-R₇₀ | -COOH | -CH(COOR₇₀)₂ |
| -Z-COOH | -Z-OC(O)-R₇₀ | -Z-COOH | -C(O)NH-R₇₀ |
| -Z-COOH | -C(O)-OR₇₀ | -Z-COOH | -Z-C(O)NH-R₇₀ |
| -Z-COOH | -Z-C(O)O-R₇₀ | -Z-COOH | -CH(COOR₇₀)₂ |
| -CH-(COOH)₂ | -Z-OC(O)-R₇₀ | -CH-(COOH)₂ | -C(O)NH-R₇₀ |
| -CH-(COOH)₂ | -C(O)-OR₇₀ | -CH-(COOH)₂ | -Z-C(O)NH-R₇₀ |
| -CH-(COOH)₂ | -Z-C(O)O-R₇₀ | -CH-(COOH)₂ | -CH(COOR₇₀)₂ |

One embodiment of the present invention relates to compound IIb 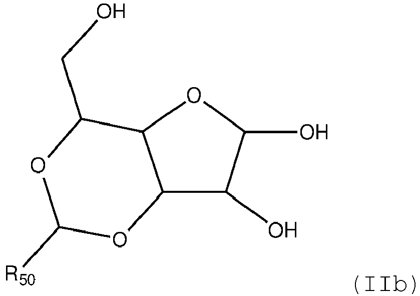 wherein R₅₀ is selected from the group consisting of -Z-OC(O)R₇₀, -COOR₇₀ and -Z-COOR₇₀, most preferably selected from the group consisting of -CH₂-OC(O)(CH₂)₄CH₃, -CH₂-OC(O)(CH₂)₅CH₃, -CH₂-OC(O)(CH₂)₆CH₃, -CH₂-OC(O)(CH₂)₇CH₃, -CH₂-OC(O)(CH₂)₈CH₃, - CH₂-OC(O)(CH₂)₉CH₃, -CH₂-OC(O)(CH₂)₁₀CH₃, -CH₂-OC(O)(CH₂)₁₁CH₃, - CH₂-OC(O)(CH₂)₁₂CH₃, -CH₂-OC(O)(CH₂)₁₃CH₃, -CH₂-OC(O)(CH₂)₁₄CH₃, - (CH₂)₂-OC(O)(CH₂)₄CH₃, -(CH₂)₂-OC(O)(CH₂)₅CH₃, -(CH₂)₂-OC(O)(CH₂)₆CH₃, -(CH₂)₂-OC(O)(CH₂)₇CH₃, -(CH₂)₂-OC(O)(CH₂)₈CH₃, - (CH₂)₂-OC(O)(CH₂)₈CH₃, -(CH₂)₂-OC(O)(CH₂)₉CH₃, -(CH₂)₂-OC(O)(CH₂)₁₀CH₃, -(CH₂)₂-OC(O)(CH₂)₁₁CH₃, -(CH₂)₂-OC(O)(CH₂)₁₂CH₃, - (CH₂)₂-OC(O)(CH₂)₁₃CH₃, -(CH₂)₂-OC(O)(CH₂)₁₂CH₃, -(CH₂)₃-OC(O)(CH₂)₄CH₃, -(CH₂)₃-OC(O)(CH₂)₅CH₃, -(CH₂)₃-OC(O)(CH₂)₆CH₃, - (CH₂)₃-OC(O)(CH₂)₇CH₃, -(CH₂)₃-OC(O)(CH₂)₈CH₃, -(CH₂)₃-OC(O)(CH₂)₈CH₃, -(CH₂)₃-OC(O)(CH₂)₉CH₃, -(CH₂)₃-OC(O)(CH₂)₁₀CH₃, - (CH₂)₃-OC(O)(CH₂)₁₁CH₃, -(CH₂)₃-OC(O)(CH₂)₁₂CH₃, -(CH₂)₃-OC(O)(CH₂)₁₃CH₃, -(CH₂)₃-OC(O)(CH₂)₁₂CH₃, -(CH₂)₄-OC(O)(CH₂)₄CH₃, - (CH₂)₄-OC(O)(CH₂)₅CH₃, -(CH₂)₄-OC(O)(CH₂)₆CH₃, -(CH₂)₄-OC(O)(CH₂)₇CH₃, -(CH₂)₄-OC(O)(CH₂)₈CH₃, -(CH₂)₄-OC(O)(CH₂)₈CH₃, - (CH₂)₄-OC(O)(CH₂)₉CH₃, -(CH₂)₄-OC(O)(CH₂)₁₀CH₃, -(CH₂)₄-OC(O)(CH₂)₁₁CH₃, -(CH₂)₄-OC(O)(CH₂)₁₂CH₃, -(CH₂)₄-OC(O)(CH₂)₁₃CH₃, - (CH₂)₄-OC(O)(CH₂)₁₂CH₃, -(CH₂)₅-OC(O)(CH₂)₄CH₃, -(CH₂)₅-OC(O)(CH₂)₅CH₃, -(CH₂)₅-OC(O)(CH₂)₆CH₃, -(CH₂)₅-OC(O)(CH₂)₇CH₃, - (CH₂)₅-OC(O)(CH₂)₈CH₃, -(CH₂)₅-OC(O)(CH₂)₈CH₃, -(CH₂)₅-OC(O)(CH₂)₉CH₃, -(CH₂)₅-OC(O)(CH₂)₁₀CH₃, -(CH₂)₅-OC(O)(CH₂)₁₁CH₃, - (CH₂)₂-OC(O)(CH₂)₁₂CH₃, -(CH₂)₅-OC(O)(CH₂)₁₃CH₃, -(CH₂)₅-OC(O)(CH₂)₁₂CH₃, -COO(CH₂)₅CH₃, -COO(CH₂)₆CH₃, -COO(CH₂)₇CH₃, - COO(CH₂)₈CH₃, -COO(CH₂)₉CH₃, -COO(CH₂)₁₀CH₃, -COO(CH₂)₁₁CH₃, - COO(CH₂)₁₂CH₃, -COO(CH₂)₁₃CH₃, -COO(CH₂)₁₄CH₃, -CH₂-C(O)O(CH₂)₄CH₃, -CH₂-C(O)O(CH₂)₅CH₃, -CH₂-C(O)O(CH₂)₆CH₃, -CH₂-C(O)O(CH₂)₇CH₃, - CH₂-C(O)O(CH₂)₈CH₃, -CH₂-C(O)O(CH₂)₉CH₃, -CH₂-C(O)O(CH₂)₁₀CH₃, - CH₂-C(O)O(CH₂)₁₁CH₃, -CH₂-C(O)O(CH₂)₁₂CH₃, -CH₂-C(O)O(CH₂)₁₃CH₃, -CH₂-C(O)O(CH₂)₁₄CH₃, -(CH₂)₂- C(O)O(CH₂)₄CH₃, -(CH₂)₂-C(O)OCH₂)₅CH₃, -(CH₂)₂- C(O)OCH₂)₆CH₃, -(CH₂)₂- C(O)OCH₂)₇CH₃, - (CH₂)₂- C(O)O(CH₂)₈CH₃, -(CH₂)₂- C(O)O(CH₂)₈CH₃, -(CH₂)₂-C(O)O(CH₂)₉CH₃, -(CH₂)₂-C(O)OCH₂)₁₀CH₃, -(CH₂)₂-C(O)O(CH₂)₁₁CH₃, - (CH₂)₂-C(O)OCH₂)₁₂CH₃, -(CH₂)₂-C(O)O(CH₂)₁₃CH₃, -(CH₂)₂-C (O)O(CH₂)₁₂CH₃, -(CH₂)₃-C(O)O(CH₂)₄CH₃, -(CH₂)₃-C(O)O(CH₂)₅CH₃, -(CH₂)₃-C(O)O(CH₂)₆CH₃, -(CH₂)₃-C(O)O(CH₂)₇CH₃, -(CH₂)₃-C(O)O(CH₂)₈CH₃, -(CH₂)₃-C(O)O(CH₂)₈CH₃, -(CH₂)₃-C(O)O(CH₂)₉CH₃, - (CH₂)₃-C(O)O(CH₂)₁₀CH₃, -(CH₂)₃-C(O)O(CH₂)₁₁CH₃, -(CH₂)₃-C(O)O (CH₂)₁₂CH₃, -(CH₂)₃-C(O)OCH₂)₁₃CH₃, -(CH₂)₃-C(O)O(CH₂)₁₂CH₃, -(CH₂)₄-C(O)O(CH₂)₄CH₃, -(CH₂)₄-C(O)O(CH₂)₅CH₃, -(CH₂)₄-C(O)O(CH₂)₆CH₃, - (CH₂)₄-C(O)O(CH₂)₇CH₃, -(CH₂)₄-C(O)O(CH₂)₈CH₃, -(CH₂)₄-C(O)O(CH₂)₈CH₃, -(CH₂)₄-C(O)O(CH₂)₉CH₃, -(CH₂)₄-C(O)O(CH₂)₁₀CH₃, - (CH₂)₄-C(O)O(CH₂)₁₁CH₃, -(CH₂)₄-C(O)O(CH₂)₁₂CH₃, -(CH₂)₄-C(O)O (CH₂)₁₃CH₃, -(CH₂)₄-C(O)O(CH₂)₁₂CH₃, -(CH₂)₅-C(O)O(CH₂)₄CH₃, -(CH₂)₅-C(O)O(CH₂)₅CH₃, -(CH₂)₅-C(O)O(CH₂)₆CH₃, -(CH₂)₅-C(O)O(CH₂)₇CH₃, - (CH₂)₅-C(O)O(CH₂)₈CH₃, -(CH₂)₅-C(O)OCH₂)₈CH₃, -(CH₂)₅-C(O)O (CH₂)₉CH₃, -(CH₂)₅-C(O)O(CH₂)₁₀CH₃, -(CH₂)₅-C(O)O(CH₂)₁₁CH₃, - (CH₂)₂-C(O)O(CH₂)₁₂CH₃, -(CH₂)₅-C(O)O(CH₂)₁₃CH₃, and -(CH₂)₅-C(O)O (CH₂)₁₂CH₃.

One embodiment of the present invention relates to compound IIc, 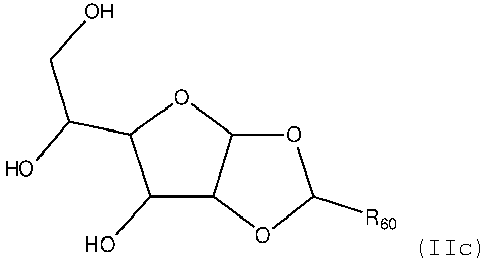 wherein R₆₀ is selected from the group consisting of -R₇₀, -Z-OC(O)-R₇₀, -C(O)-OR₇₀, -Z-C(O)O-R₇₀, -C(O)NH-R₇₀, -Z-C(O)NH-R₇₀ and -CH(COOR₇₀)₂, preferably selected from the group consisting of -Z-C(O)-OR₇₀, -C(O)-OR₇₀ and -R₇₀, and most preferably selected from the group consisting of -C(O)-O(CH₂)₄CH₃, -C(O)-O(CH₂)₅CH₃, -C(O)-O(CH₂)₆CH₃, -C(O)-O(CH₂)₇CH₃, -C(O)-O(CH₂)₈CH₃, -C(O)-O(CH₂)₉CH₃, -C(O)-O(CH₂)₁₀CH₃, -C(O)-O(CH₂)₁₁CH₃, -C(O)-O(CH₂)₁₂CH₃, -C(O)-O(CH₂)₁₃CH₃, -C(O)-O(CH₂)₁₄CH₃, -CH₂-C(O)-O(CH₂)₄CH₃, -CH₂-C(O)-O(CH₂)₅CH₃, -CH₂-C(O)-O(CH₂)₆CH₃, -CH₂-C(O)-O(CH₂)₇CH₃, -CH₂-C(O)-O(CH₂)₈CH₃, -CH₂-C(O)-O(CH₂)₉CH₃, - CH₂-C(O)-O(CH₂)₁₀CH₃, -CH₂-C(O)-O(CH₂)₁₁CH₃, -CH₂-C(O) - O(CH₂)₁₂CH₃, -CH₂-C(O)-O(CH₂)₁₃CH₃, -CH₂-C(O)-O(CH₂)₁₄CH₃, - (CH₂)₂-C(O)-O(CH₂)₄CH₃, -(CH₂)₂-C(O)-O(CH₂)₅CH₃, -(CH₂)₂-C(O)-O(CH₂)₆CH₃, -(CH₂)₂-C(O)-O(CH₂)₇CH₃, -(CH₂)₂-C(O)-O(CH₂)₈CH₃, - (CH₂)₂-C(O)-O(CH₂)₉CH₃, -(CH₂)₂-C(O)-O(CH₂)₁₀CH₃, -(CH₂)₂-C(O)-O(CH₂)₁₁CH₃, -(CH₂)₂-C(O)-O(CH₂)₁₂CH₃, -(CH₂)₂-C(O)-O(CH₂)₁₃CH₃, - (CH₂)₂-C(O)-O(CH₂)₁₄CH₃, -(CH₂)₃-C(O)-O(CH₂)₄CH₃, -(CH₂)₃-C(O)-O(CH₂)₅CH₃, -(CH₂)₃-C(O)-O(CH₂)₆CH₃, -(CH₂)₃-C(O)-O(CH₂)₇CH₃, - (CH₂)₃-C(O)-O(CH₂)₈CH₃, -(CH₂)₃-C(O)-O(CH₂)₉CH₃, -(CH₂)₃-C(O)-O(CH₂)₁₀CH₃, -(CH₂)₃-C(O)-O(CH₂)₁₁CH₃, -(CH₂)₃-C(O)-O(CH₂)₁₂CH₃, - (CH₂)₃-C(O)-O(CH₂)₁₃CH₃, -(CH₂)₃-C(O)-O(CH₂)₁₄CH₃, -(CH₂)₄CH₃, - (CH₂)₅CH₃, -(CH₂)₆CH₃, -(CH₂)₇CH₃, -(CH₂)₈CH₃, -(CH₂)₉CH₃, - (CH₂)₁₀CH₃, - (CH₂)₁₁CH₃, -(CH₂)₁₂CH₃, - (CH₂)₁₃CH₃, and - (CH₂)₁₄CH₃.

One embodiment of the present invention relates to compound IId, 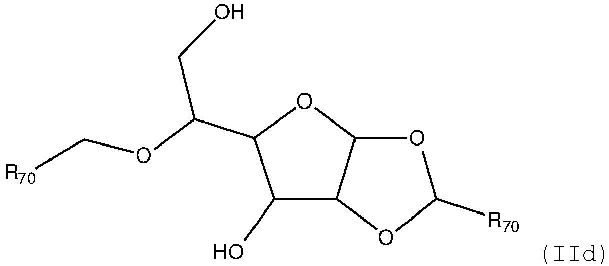 wherein R₇₀ is selected from the group consisting of -(CH₂)₄CH₃, -(CH₂)₅CH₃, -(CH₂)₆CH₃, -(CH₂)₇CH₃, -(CH₂)₈CH₃, -(CH₂)₉CH₃, - (CH₂)₁₀CH₃, -(CH₂)₁₁CH₃, -(CH₂)₁₂CH₃, -(CH₂)₁₃CH₃, and -(CH₂)₁₄CH₃.

One embodiment of the present invention relates to compound IIe, 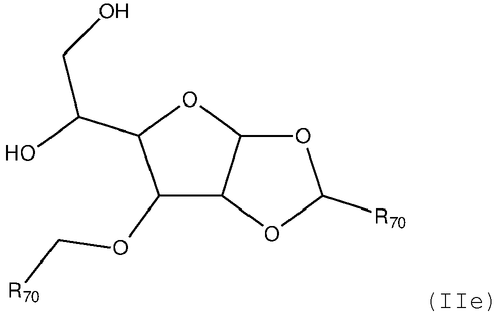 wherein R₇₀ is selected from the group consisting of - (CH₂)₄CH₃, -(CH₂)₅CH₃, -(CH₂)₆CH₃, -(CH₂)₇CH₃, -(CH₂)₈CH₃, -(CH₂)₉CH₃, - (CH₂)₁₀CH₃, -(CH₂)₁₁CH₃, -(CH₂)₁₂CH₃, -(CH₂)₁₃CH₃, and -(CH₂)₁₄CH₃.

One embodiment of the present invention relates to compound IIIa, 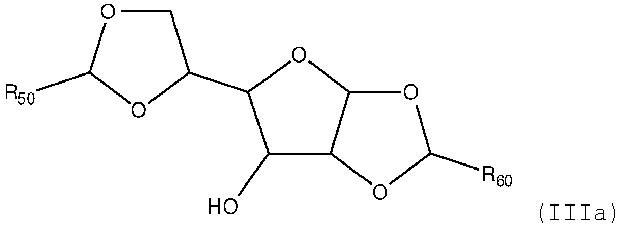 wherein R₅₀ and R₆₀ are as defined below:

| R₅₀ | R₆₀ | R₅₀ | R₆₀ |
|---|---|---|---|
| -Z-OC(O)-R₇₀ | -Z-OH | -C(O)NH-R₇₀ | -Z-OH |
| -C(O)-OR₇₀ | -Z-OH | -Z-C(O)NH-R₇₀ | -Z-OH |
| -Z-C(O)O-R₇₀ | -Z-OH | -CH(COOR₇₀)₂ | -Z-OH |
| -Z-OC(O)-R₇₀ | -COOH | -C(O)NH-R₇₀ | -COOH |
| -C(O)-OR₇₀ | -COOH | -Z-C(O)NH-R₇₀ | -COOH |
| -Z-C(O)O-R₇₀ | -COOH | -CH(COOR₇₀)₂ | -COOH |
| -Z-OC(O)-R₇₀ | -Z-COOH | -C(O)NH-R₇₀ | -Z-COOH |
| -C(O)-OR₇₀ | -Z-COOH | -Z-C(O)NH-R₇₀ | -Z-COOH |
| -Z-C(O)O-R₇₀ | -Z-COOH | -CH(COOR₇₀)₂ | -Z-COOH |
| -Z-OC(O)-R₇₀ | -CH-(COOH)₂ | -C(O)NH-R₇₀ | -CH-(COOH)₂ |
| -C(O)-OR₇₀ | -CH-(COOH)₂ | -Z-C(O)NH-R₇₀ | -CH-(COOH)₂ |
| -Z-C(O)O-R₇₀ | -CH-(COOH)₂ | -CH(COOR₇₀)₂ | -CH-(COOH)₂ |
| -Z-OH | -Z-OC(O)-R₇₀ | -Z-OH | -C(O)NH-R₇₀ |
| -Z-OH | -C(O)-OR₇₀ | -Z-OH | -Z-C(O)NH-R₇₀ |
| -Z-OH | -Z-C(O)O-R₇₀ | -Z-OH | -CH(COOR₇₀)₂ |
| -COOH | -Z-OC(O)-R₇₀ | -COOH | -C(O)NH-R₇₀ |
| -COOH | -C(O)-OR₇₀ | -COOH | -Z-C(O)NH-R₇₀ |
| -COOH | -Z-C(O)O-R₇₀ | -COOH | -CH(COOR₇₀)₂ |
| -Z-COOH | -Z-OC(O)-R₇₀ | -Z-COOH | -C(O)NH-R₇₀ |
| -Z-COOH | -C(O)-OR₇₀ | -Z-COOH | -Z-C(O)NH-R₇₀ |
| -Z-COOH | -Z-C(O)O-R₇₀ | -Z-COOH | -CH(COOR₇₀)₂ |
| -CH-(COOH)₂ | -Z-OC(O)-R₇₀ | -CH-(COOH)₂ | -C(O)NH-R₇₀ |
| -CH-(COOH)₂ | -C(O)-OR₇₀ | -CH-(COOH)₂ | -Z-C(O)NH-R₇₀ |
| -CH-(COOH)₂ | -Z-C(O)O-R₇₀ | -CH-(COOH)₂ | -CH (COOR₇₀)₂ |

One embodiment of the present invention relates to compound IIIb, 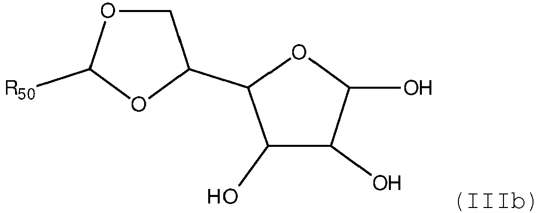 wherein R₅₀ is selected from the group consisting of -R₇₀, -Z-OC(O)R₇₀, -COOR₇₀ and -Z-COOR₇₀, most preferably selected from the group consisting of -CH₂-OC(O) (CH₂)₄CH₃, -CH₂-OC(O)(CH₂)₅CH₃, -CH₂-OC(O)(CH₂)₆CH₃, -CH₂-OC(O)(CH₂)₇CH₃, -CH₂-OC(O)(CH₂)₈CH₃, - CH₂-OC(O)(CH₂)₉CH₃, -CH₂-OC(O)(CH₂)₁₀CH₃, -CH₂-OC(O)(CH₂)₁₁CH₃, - CH₂-OC(O)(CH₂)₁₂CH₃, -CH₂-OC(O)(CH₂)₁₃CH₃, -CH₂-OC(O)(CH₂)₁₄CH₃, - (CH₂)₂-OC(O)(CH₂)₄CH₃, -(CH₂)₂-OC(O)(CH₂)₅CH₃, -(CH₂)₂-OC(O)(CH₂)₆CH₃, - (CH₂)₂-OC(O)(CH₂)₇CH₃, - (CH₂)₂-OC(O)(CH₂)₈CH₃, - (CH₂)₂-OC(O)(CH₂)₈CH₃, - (CH₂)₂-OC(O)(CH₂)₉CH₃, - (CH₂)₂-OC(O)(CH₂)₁₀CH₃, - (CH₂)₂-OC(O)(CH₂)₁₁CH₃, - (CH₂)₂-OC(O)(CH₂)₁₂CH₃, - (CH₂)₂-OC(O)(CH₂)₁₃CH₃, - (CH₂)₂-OC(O)(CH₂)₁₂CH₃, - (CH₂)₃-OC(O)(CH₂)₄CH₃, -(CH₂)₃-OC(O)(CH₂)₅CH₃, - (CH₂)₃-OC(O)(CH₂)₆CH₃, - (CH₂)₃-OC(O)(CH₂)₇CH₃, - (CH₂)₃-OC(O)(CH₂)₈CH₃, - (CH₂)₃-OC(O)(CH₂)₈CH₃, - (CH₂)₃-OC(O)(CH₂)₉CH₃, - (CH₂)₃-OC(O)(CH₂)₁₀CH₃, - (CH₂)₃-OC(O)(CH₂)₁₁CH₃, - (CH₂)₃-OC(O)(CH₂)₁₂CH₃, -(CH₂)₃-OC(O)(CH₂)₁₃CH₃, - (CH₂)₃-OC(O)(CH₂)₁₂CH₃, -(CH₂)₄-OC(O)(CH₂)₄CH₃, - (CH₂)₄-OC(O)(CH₂)₅CH₃, - (CH₂)₄-OC(O)(CH₂)₆CH₃, -(CH₂)₄-OC(O)(CH₂)₇CH₃, - (CH₂)₄-OC(O)(CH₂)₈CH₃, - (CH₂)₄-OC(O)(CH₂)₈CH₃, - (CH₂)₄-OC(O)(CH₂)₉CH₃, - (CH₂)₄-OC(O)(CH₂)₁₀CH₃, - (CH₂)₄-OC(O)(CH₂)₁₁CH₃, - (CH₂)₄-OC(O)(CH₂)₁₂CH₃, - (CH₂)₄-OC(O)(CH₂)₁₃CH₃, - (CH₂)₄-OC(O)(CH₂)₁₂CH₃, - (CH₂)₅-OC(O)(CH₂)₄CH₃, - (CH₂)₅-OC(O)(CH₂)₅CH₃, - (CH₂)₅-OC(O)(CH₂)₆CH₃, - (CH₂)₅-OC(O)(CH₂)₇CH₃, - (CH₂)₅-OC(O)(CH₂)₈CH₃, - (CH₂)₅-OC(O)(CH₂)₈CH₃, -(CH₂)₅-OC(O)(CH₂)₉CH₃, - (CH₂)₅-OC(O)(CH₂)₁₀CH₃, - (CH₂)₅-OC(O)(CH₂)₁₁CH₃, - (CH₂)₂-OC(O)(CH₂)₁₂CH₃, - (CH₂)₅-OC(O)(CH₂)₁₃CH₃, -(CH₂)₅-OC(O)(CH₂)₁₂CH₃, -COO (CH₂)₅CH₃, -COO (CH₂)₆CH₃, -COO (CH₂)₇CH₃, - COO (CH₂)₈CH₃, -COO (CH₂)₉CH₃, -COO(CH₂)₁₀CH₃, -COO(CH₂)₁₁CH₃, - COO (CH₂)₁₂CH₃, -COO(CH₂)₁₃CH₃, -COO(CH₂)₁₄CH₃, -CH₂-C(O)O(CH₂)₄CH₃, -CH₂-C(O)O(CH₂)₅CH₃, -CH₂-C(O)O(CH₂)₆CH₃, -CH₂-C(O)O(CH₂)₇CH₃, - CH₂-C(O)O(CH₂)₈CH₃, -CH₂-C(O)O(CH₂)₉CH₃, -CH₂- C(O)O(CH₂)₁₀CH₃, - CH₂- C(O)O(CH₂)₁₁CH₃, -CH₂- C (O)O(CH₂)₁₂CH₃, -CH₂- C (O)O(CH₂)₁₃CH₃, -CH₂-C(O)O(CH₂)₁₄CH₃, -(CH₂)₂- C(O)O(CH₂)₄CH₃, -(CH₂)₂-C(O)OCH₂)₅CH₃, -(CH₂)₂- C(O)OCH₂)₆CH₃, - (CH₂)₂- C(O)OCH₂)₇CH₃, - (CH₂)₂- C(O)O(CH₂)₈CH₃, - (CH₂)₂- C(O)O(CH₂)₈CH₃, - (CH₂)₂-C(O)O(CH₂)₉CH₃, - (CH₂)₂-C(O)OCH₂)₁₀CH₃, - (CH₂)₂-C(O)O(CH₂)₁₁CH₃, - (CH₂)₂-C(O)OCH₂)₁₂CH₃, -(CH₂)₂-C(O)O(CH₂)₁₃CH₃, -(CH₂)₂-C(O)O(CH₂)₁₂CH₃, -(CH₂)₃- C(O)O(CH₂)₄CH₃, - (CH₂)₃-C(O)O(CH₂)₅CH₃, -(CH₂)₃-C(O)O(CH₂)₆CH₃, - (CH₂)₃-C(O)O(CH₂)₇CH₃, - (CH₂)₃-C(O)O(CH₂)₈CH₃, - (CH₂)₃-C(O)O(CH₂)₈CH₃, - (CH₂)₃- C(O)O(CH₂)₉CH₃, - (CH₂)₃-C(O)O(CH₂)₁₀CH₃, - (CH₂)₃-C(O)O(CH₂)₁₁CH₃, - (CH₂)₃-C(O)O (CH₂)₁₂CH₃, - (CH₂)₃-C(O)OCH₂)₁₃CH₃, - (CH₂)₃-C(O)O(CH₂)₁₂CH₃, - (CH₂)₄-C(O)O(CH₂)₄CH₃, - (CH₂)₄-C(O)O(CH₂)₅CH₃, - (CH₂)₄-C(O)O(CH₂)₆CH₃, - (CH₂)₄-C(O)O(CH₂)₇CH₃, - (CH₂)₄-C(O)O(CH₂)₈CH₃, - (CH₂)₄-C(O)O(CH₂)₈CH₃, - (CH₂)₄-C(O)O(CH₂)₉CH₃, - (CH₂)₄-C(O)O(CH₂)₁₀CH₃, - (CH₂)₄-C(O)O(CH₂)₁₁CH₃, - (CH₂)₄-C(O)O(CH₂)₁₂CH₃, - (CH₂)₄-C(O)O (CH₂)₁₃CH₃, - (CH₂)₄-C(O)O(CH₂)₁₂CH₃, - (CH₂)₅-C (O)O(CH₂)₄CH₃, - (CH₂)₅-C(O)O(CH₂)₅CH₃, - (CH₂)₅-C (O)O(CH₂)₆CH₃, - (CH₂)₅-C(O)O(CH₂)₇CH₃, - (CH₂)₅-C(O)O(CH₂)₈CH₃, - (CH₂)₅-C(O)OCH₂)₈CH₃, - (CH₂)₅-C(O)O (CH₂)₉CH₃, - (CH₂)₅-C(O)O(CH₂)₁₀CH₃, - (CH₂)₅-C(O)O(CH₂)₁₁CH₃, - (CH₂)₂-C(O)O(CH₂)₁₂CH₃, - (CH₂)₅-C(O)O(CH₂)₁₃CH₃ and - (CH₂)₅-C(O)O (CH₂)₁₂CH₃.

One embodiment of the present invention relates to compound IIIc, 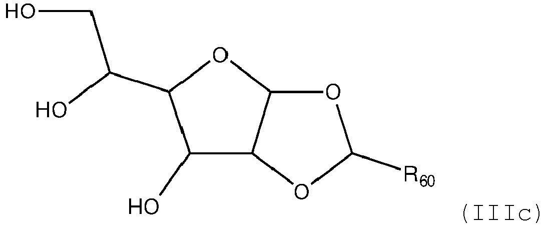 wherein R₆₀ is selected from the group consisting of -R₇₀, -Z-OC(O)-R₇₀, -C(O)-OR₇₀, -Z-C(O)O-R₇₀, -C(O)NH-R₇₀, -Z-C(O)NH-R₇₀, -CH(COOR₇₀)₂, preferably selected from the group consisting of -Z-C(O)-OR₇₀, -C(O)-OR₇₀ and -R₇₀, and most preferably selected from the group consisting of -C(O)-O(CH₂)₄CH₃, -C(O)-O(CH₂)₅CH₃, -C(O)-O(CH₂)₆CH₃, -C(O)-O(CH₂)₇CH₃, -C(O)-O(CH₂)₈CH₃, -C(O)-O(CH₂)₉CH₃, -C(O)-O(CH₂)₁₀CH₃, -C(O)-O(CH₂)₁₁CH₃, -C(O)-O(CH₂)₁₂CH₃, -C(O)-O(CH₂)₁₃CH₃, -C(O)-O(CH₂)₁₄CH₃, -CH₂-C(O)-O(CH₂)₄CH₃, -CH₂-C(O)-O(CH₂)₅CH₃, -CH₂-C(O)-O(CH₂)₆CH₃, -CH₂-C(O)-O(CH₂)₇CH₃, -CH₂-C(O)-O(CH₂)₈CH₃, -CH₂-C(O)-O(CH₂)₉CH₃, - CH₂-C(O)-O(CH₂)₁₀CH₃, -CH₂-C(O)-O(CH₂)₁₁CH₃, -CH₂-C(O)-O(CH₂)₁₂CH₃, -CH₂-C(O)-O(CH₂)₁₃CH₃, -CH₂-C(O)-O(CH₂)₁₄CH₃, - (CH₂)₂-C(O)-O(CH₂)₄CH₃, - (CH₂)₂-C(O)-O(CH₂)₅CH₃, - (CH₂)₂-C(O)-O(CH₂)₆CH₃, - (CH₂)₂-C(O)-O(CH₂)₇CH₃, - (CH₂)₂-C(O)-O(CH₂)₈CH₃, - (CH₂)₂-C(O)-O(CH₂)₉CH₃, - (CH₂)₂-C(O)-O(CH₂)₁₀CH₃, - (CH₂)₂-C(O)-O(CH₂)₁₁CH₃, - (CH₂)₂-C(O)-O(CH₂)₁₂CH₃, - (CH₂)₂-C(O)-O(CH₂)₁₃CH₃, - (CH₂)₂-C(O)-O(CH₂)₁₄CH₃, - (CH₂)₃-C(O)-O(CH₂)₄CH₃, - (CH₂)₃-C(O)-O(CH₂)₅CH₃, - (CH₂)₃-C(O)-O(CH₂)₆CH₃, - (CH₂)₃-C(O)-O(CH₂)₇CH₃, - (CH₂)₃-C(O)-O(CH₂)₈CH₃, - (CH₂)₃-C(O)-O(CH₂)₉CH₃, - (CH₂)₃-C(O)-O(CH₂)₁₀CH₃, - (CH₂)₃-C(O)-O(CH₂)₁₁CH₃, - (CH₂)₃-C(O)-O(CH₂)₁₂CH₃, - (CH₂)₃-C(O)-O(CH₂)₁₃CH₃, - (CH₂)₃-C(O)-O(CH₂)₁₄CH₃, - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, and - (CH₂)₁₄CH₃.

One embodiment of the present invention relates to compound IIId, 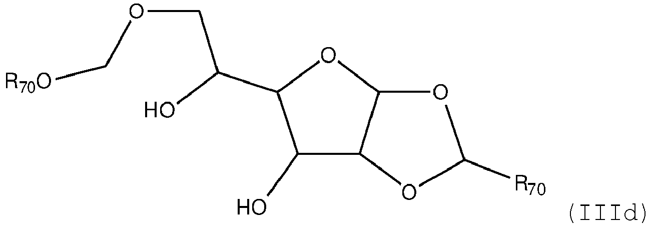 wherein R₇₀ is selected from the group consisting of - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, and -(CH₂)₁₄CH₃.

One embodiment of the present invention relates to compound IIIe, 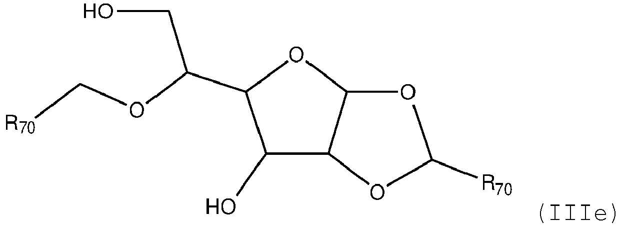 wherein R₇₀ is selected from the group consisting of - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, and - (CH₂)₁₄CH₃.

One embodiment of the present invention relates to a compound of the general formula (I), (II) and (III) wherein R₁₁ and R₁₂ or R₂₁ and R₂₂ or R₃₁ and R₃₂ are both hydrogen or form together with CHR₅₀ a cyclic moiety, or wherein R₁₃ and R₁₄ or R₂₃ and R₂₄ or R₃₃ and R₃₄ are both hydrogen or form together with CHR₆₀ a cyclic moiety, with the proviso that not all of R₁₁, R₁₂, R₁₃ and R₁₄ or R₂₁, R₂₂, R₂₃ and R₂₄ or R₃₁, R₃₂, R₃₃ and R₃₄ are hydrogen, wherein R₅₀ or R₆₀ are a linear C₁ to C₁₀ alkyl. Preferably, said compound is selected from the group consisting of compound la, Ib, Ic, IIa, IIb, IIc, IIIa, IIIb and IIIc, wherein R₅₀ or R₆₀ are a linear C₁ to C₁₀ alkyl, most preferably C₅ to C₁₀ alkyl.

One embodiment of the present invention relates to a compound of the general formula (I), (II) and (III) wherein R₁₁ and R₁₂ or R₂₁ and R₂₂ or R₃₁ and R₃₂ form together with CHR₅₀ a cyclic moiety and wherein R₁₃ and R₁₄ or R₂₃ and R₂₄ or R₃₃ and R₃₄ form together with CHR₆₀ a cyclic moiety, and R₅₀ and R₆₀ are different and are selected from the group consisting of -COOH and - C(O)NH-R₇₀, and wherein -R₇₀ is preferably a linear or branched C₁ to C₂₀ alkyl, more preferably a linear C₅ to C₁₅ alkyl and most preferably a linear C₁₀ to C₁₅ alkyl. Preferably, said compound is selected from the group consisting of compound Ia, IIa and IIIa, wherein R₅₀ and R₆₀ are defined as follows:

| R₅₀ | R₆₀ | R₅₀ | R₆₀ |
|---|---|---|---|
| -COOH | -C(O)NH-CH₃ | -C(O)NH-CH₃ | -COOH |
| -COOH | -C(O)NH-(CH₂)-CH₃ | -C(O)NH-(CH₂)-CH₃ | -COOH |
| -COOH | -C(O)NH-(CH₂)₂-CH₃ | -C(O)NH-(CH₂)₂-CH₃ | -COOH |
| -COOH | -C(O)NH-(CH₂)₃-CH₃ | -C(O)NH-(CH₂)₃-CH₃ | -COOH |
| -COOH | -C(O)NH-(CH₂)₄-CH₃ | -C(O)NH-(CH₂)₄-CH₃ | -COOH |
| -COOH | -C(O)NH-(CH₂)₅-CH₃ | -C(O)NH-(CH₂)₅-CH₃ | -COOH |
| -COOH | -C(O)NH-(CH₂)₆-CH₃ | -C(O)NH-(CH₂)₆-CH₃ | -COOH |
| -COOH | -C(O)NH-(CH₂)₇-CH₃ | -C(O)NH-(CH₂)₇-CH₃ | -COOH |
| -COOH | -C(O)NH-(CH₂)₈-CH₃ | -C(O)NH-(CH₂)₈-CH₃ | -COOH |
| -COOH | -C(O)NH-(CH₂)₉-CH₃ | -C(O)NH-(CH₂)₉-CH₃ | -COOH |
| -COOH | -C(O)NH-(CH₂)₁₀-CH₃ | -C(O)NH-(CH₂)₁₀-CH₃ | -COOH |
| -COOH | -C(O)NH-(CH₂)₁₁-CH₃ | -C(O)NH-(CH₂)₁₁-CH₃ | -COOH |
| -COOH | -C(O)NH-(CH₂)₁₂-CH₃ | -C(O)NH-(CH₂)₁₂-CH₃ | -COOH |
| -COOH | -C(O)NH-(CH₂)₁₃-CH₃ | -C(O)NH-(CH₂)₁₃-CH₃ | -COOH |
| -COOH | -C(O)NH-(CH₂)₁₄-CH₃ | -C(O)NH-(CH₂)₁₄-CH₃ | -COOH |
| -COOH | -C(O)NH-(CH₂)₁₅-CH₃ | -C(O)NH-(CH₂)₁₅-CH₃ | -COOH |
| -COOH | -C(O)NH-(CH₂)₁₆-CH₃ | -C(O)NH-(CH₂)₁₆-CH₃ | -COOH |
| -COOH | -C(O)NH-(CH₂)₁₇-CH₃ | -C(O)NH-(CH₂)₁₇-CH₃ | -COOH |
| -COOH | -C(O)NH-(CH₂)₁₈-CH₃ | -C(O)NH-(CH₂)₁₈-CH₃ | -COOH |
| -COOH | -C(O)NH-(CH₂)₁₉-CH₃ | -C(O)NH-(CH₂)₁₉-CH₃ | -COOH |

One embodiment of the present invention relates to a compound of the general formula (I), (II) and (III), preferably a compound of formula Ia, Ib, Ic, IIa, IIb, IIc, IIIa, IIIb, and IIIc wherein one of R₅₀ and R₆₀ is selected from the group consisting of -Z-OC(O)R₇₀, -COOR₇₀ and -Z-COOR₇₀ and the other, if present, is selected from the group consisting of -Z-OH, - COOH and -Z-COOH, and Z is a linear or branched C₁ to C₁₀ alkyl, preferably a linear C₁ to C₅ alkyl, most preferably a linear C₁ to C₃ alkyl, and wherein -R₇₀ is a linear or branched C₁ to C₂₀ alkyl, preferably a linear C₅ to C₁₅ alkyl, most preferably a linear C₁₀ to C₁₅ alkyl.

One embodiment of the present invention relates to a compound of the general formula (I), (II) and (III), preferably a compound of formula Ia, Ib, Ic, IIa, IIb, IIc, IIIa, IIIb, and IIIc, wherein R₅₀ or R₆₀ is selected from the group consisting of -C(O)NH-R₇₀ and Z-C(O)NH-R₇₀ and R₇₀ is a linear or branched C₁ to C₂₀ alkyl, preferably a linear C₅ to C₁₅ alkyl, most preferably a linear C₁₀ to C₁₅ alkyl. Said compounds are resistant towards the hydrolysis in alkaline media and provide antistatic properties. In addition, they are usually less aggressive than compounds carrying a sulphonate-group. Further they do not over-strip, i.e., they do not degrease to leave an excessively dry or 'squeaky' feel the skin or hair. Accordingly, said surfactants are especially preferred for surfactant-assisted synthesis of nanoparticles, oil-recovery with surfactant flooding, foam boosters and laundry applications. In addition, they are particularly useful as surfactants in oil recovery, in assisted synthesis of nanoparticle, as foam boosters and additives in cleaning formulations for laundry applications. In particular, it was found that said compounds provide low interfacial tensions and low microemulsion viscosities.

In one embodiment of the present invention relates to a compound of the general formula (I), (II) and (III) preferably a compound of formula la, Ib, Ic, IIa, IIb, IIc, IIIa, IIIb, and IIIc, wherein R₅₀ or R₆₀ is selected from the group consisting of Z-SO₃⁻ and Z-OH, preferably Z-SO₃⁻, and -Z is preferably a linear or branched C₁ to C₁₀ alkyl, more preferably a linear C₁ to C₅ alkyl and most preferably a linear C₁ to C₃ alkyl. Most preferably said compound is selected from the group consisting of compound la, IIa and IIIa, wherein R₅₀ is Z-OH and R₆₀ is Z-SO₃⁻ and Z is preferably a linear or branched C₁ to C₁₀ alkyl, more preferably a linear C₁ to C₅ alkyl and most preferably a linear C₁ to C₃ alkyl. Said compounds are more labile than other surfactants, but they provide an excellent detergency and stable foamability. They are especially preferred as foaming agents and as detergents. Preferably, said compounds are used in shampoos as foaming agent or detergent. Said shampoos contain significant proportions of said compounds in an aqueous medium which has preferably about a neutral pH. The shampoo has most preferably a pH in the range of 6.5 to 7.5, more preferably 6.8 to 7.3. Such shampoos have excellent foaming properties and a good rinsability. Furthermore, the foams provide a good stability.

The compounds of the present invention can be used as surfactants, preferably as detergents and additives in cleaning products.

The present invention also encompasses a composition containing the compounds of the present invention and significant amounts of water. The inclusion of higher water amount is beneficial to incorporate more hydrophilic ingredients into the composition.

In one embodiment of the present invention the compounds are used as emulsifiers, foam stabilizers, wetting agents, emollients in cosmetic products, surfactants in food products, as anti-spattering agents for frying or as surfactant in pharmaceutical products.

The compounds of the present invention can be easily obtained by the following reaction steps:

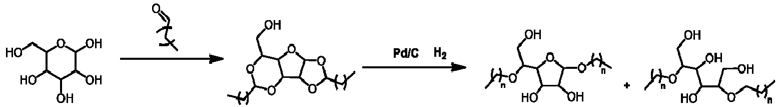

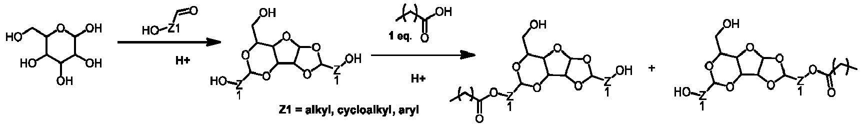

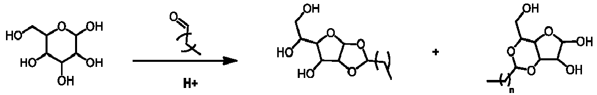

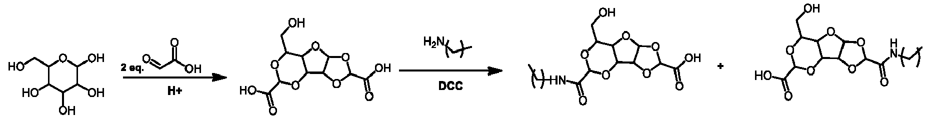

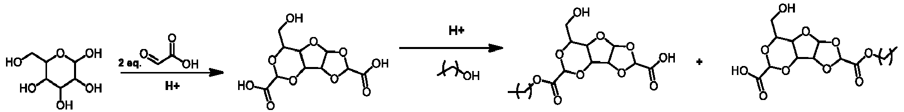

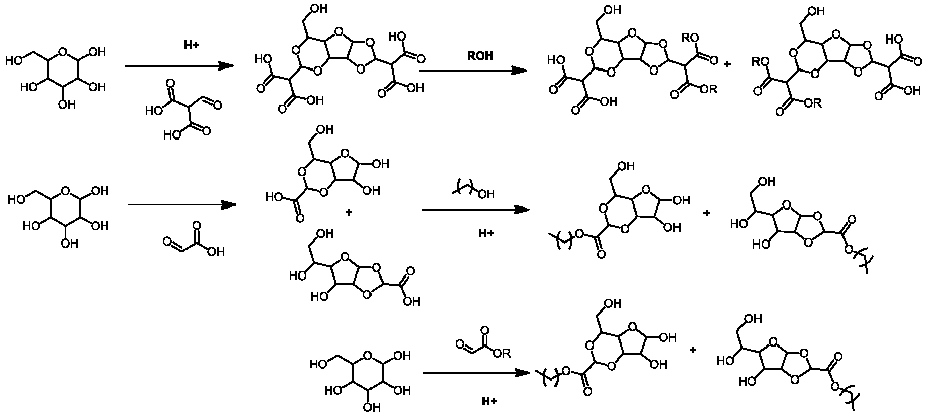

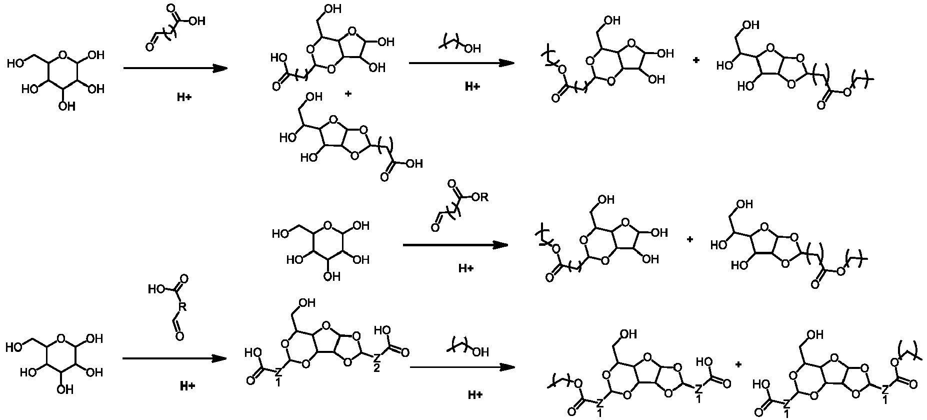

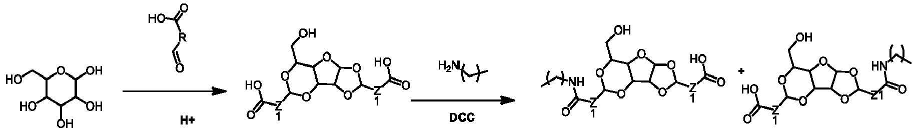

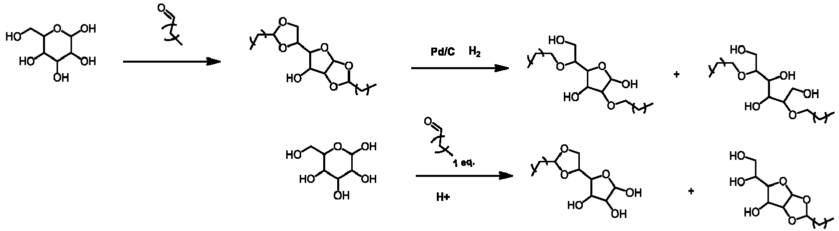

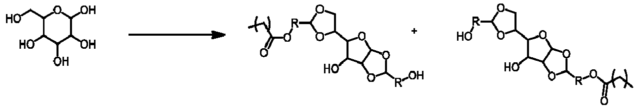

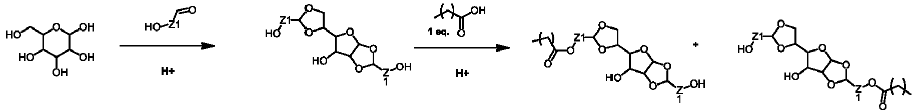

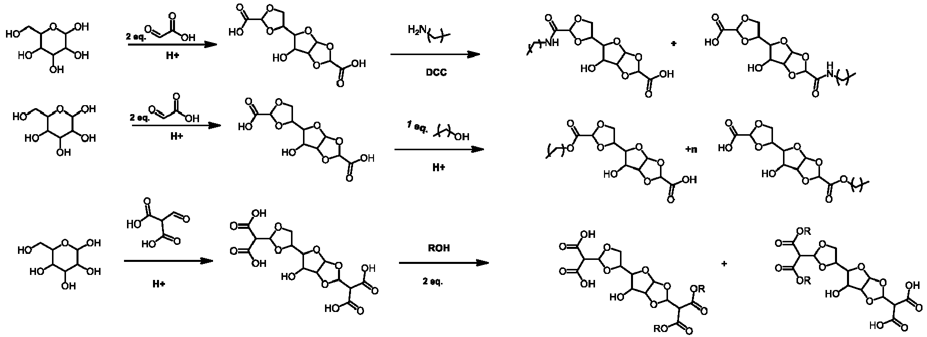

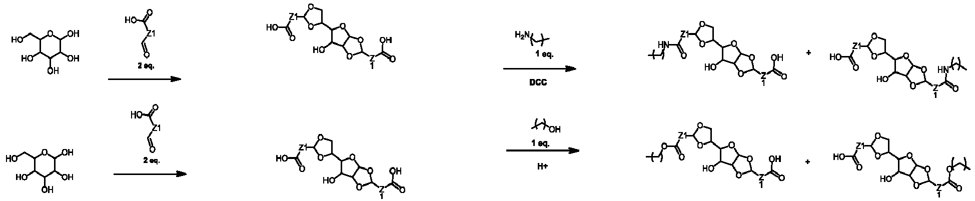

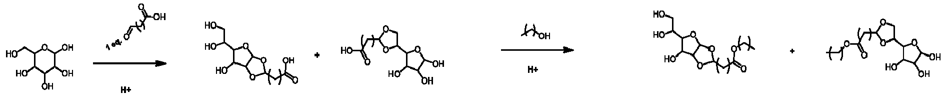

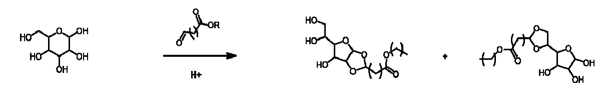

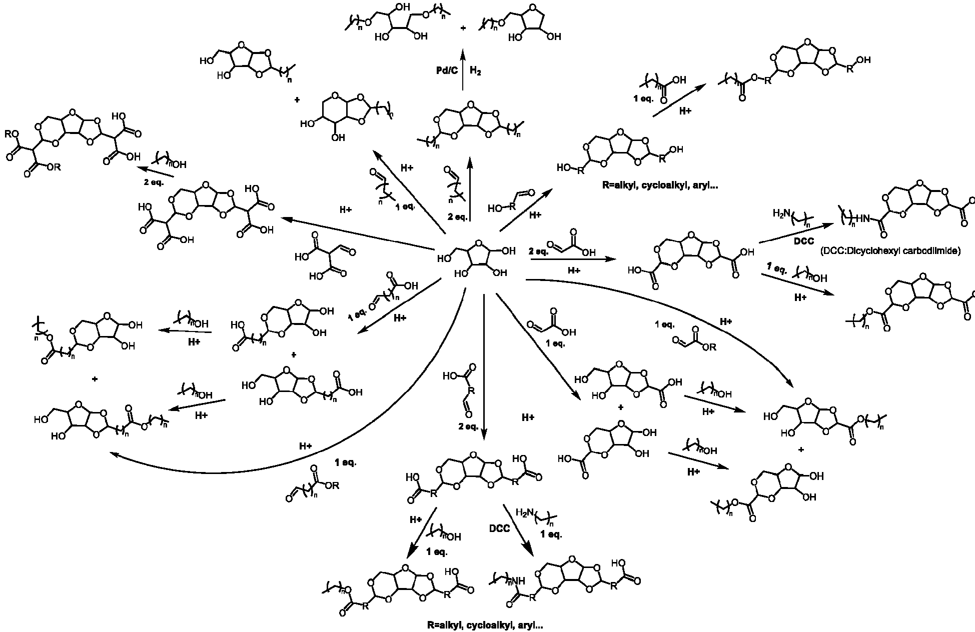

### DCC: Dicyclohexyl carbodiimide

The present invention further relates to a compound of the general formula (IV), 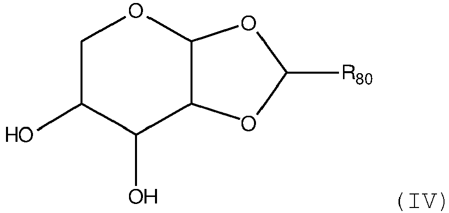 wherein R₈₀ is selected from the group consisting of a linear or branched C₁ to C₂₀ alkyl, (C₁ to C₁₀) -alkyloxy- (C₁ to C₁₀)-alkyl, C₂ to C₁₀ alkenyl, C₆ to C₁₂ aryl, C₃ to C₁₀ cycloalkyl, cycloalkylalkyl and cycloalkylalkenyl.

One embodiment of the present invention relates to compound (IV) wherein R₈₀ is selected from the group consisting of - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, and - (CH₂)₁₄CH₃, preferably selected from the group consisting of - (CH₂)₆CH₃, - (CH₂)₇CH₃, - (CH₂)₈CH₃, - (CH₂)₉CH₃, and - (CH₂)₁₀CH₃, more preferably selected from the group consisting of - (CH₂)₆CH₃, - (CH₂)₈CH₃ and - (CH₂)₁₀CH₃.

One embodiment of the present invention relates to compound (IV),

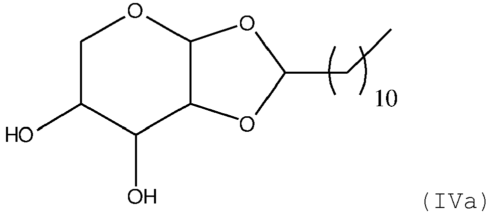

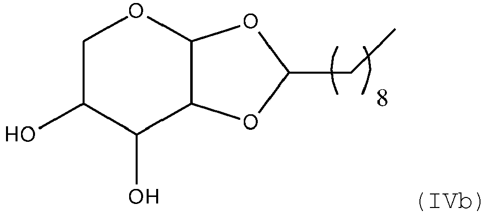

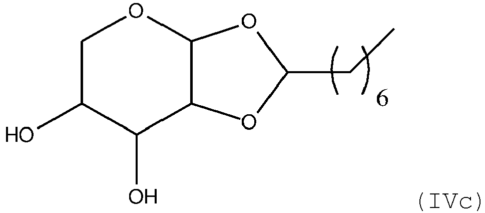

The compound of the general formula (IV) of the present invention can be used as surfactants, preferably as emulsifiers, foam stabilizers, wetting agents, emollients in cosmetic products, surfactants in food products, as anti-spattering agents for frying or as surfactant in pharmaceutical products.

The present invention further relates to a compound of the general formula (V), 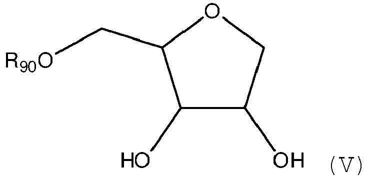 wherein R₉₀ is selected from the group consisting of a linear or branched C₁ to C₂₀ alkyl, (C₁ to C₁₀) -alkyloxy- (C₁ to C₁₀)-alkyl, C₂ to C₁₀ alkenyl, C₆ to C₁₂ aryl, C₃ to C₁₀ cycloalkyl, cycloalkylalkyl and cycloalkylalkenyl.

One embodiment of the present invention relates to compound (IV) wherein R₉₀ is selected from the group consisting of - (CH₂)₄CH₃, - (CH₂)₅CH₃, - (CH₂)₆CH₃, - (CH₂)₇CH₃, - (CH₂)₈CH₃, - (CH₂)₉CH₃, - (CH₂)₁₀CH₃, - (CH₂)₁₁CH₃, - (CH₂)₁₂CH₃, - (CH₂)₁₃CH₃, and - (CH₂)₁₄CH₃, preferably selected from the group consisting of - (CH₂)₁₁CH_{3.}

One embodiment of the present invention relates to compound (Va),

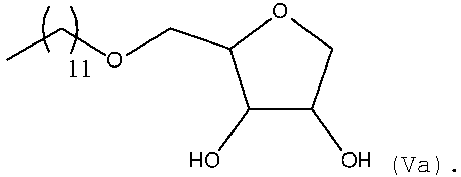

The compound of the general formula (V) of the present invention can be used as surfactants, preferably as emulsifiers, foam stabilizers, wetting agents, emollients in cosmetic products, surfactants in food products, as anti-spattering agents for frying or as surfactant in pharmaceutical products.

### Examples:

### Synthesis of docosanol-substituted diglyoxylic acid xylose (2,3) and characterization

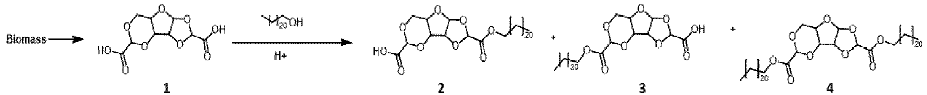

The synthesis of diglyoxylic acid-xylose (1) from xylose and biomass is known to the skilled person. Docosanol (0.56 g, 1.7 mmol) dissolved in 25 ml of chloroform and diglyoxylic acid-xylose (1) (0.5 g, 1.9 mmol) dissolved in 15 ml of 1,4-dioxane were mixed in 100 ml round-bottom flask. Sulfuric acid (98% pure, 200 mkl) was added to the reaction mixture and the reaction mixture was heated at 65°C for 24h to obtain slightly pink solution. The reaction mixture was analyzed by HPLC (C18 column, isopropanol/methanol 1:9 mobile phase) and TLC (hexane/ethyl acetate 1:8), and one product was identified. The product is now subject to purification and further identification.

### Emulsion stability measurements

The emulsions were prepared by mixing 1ml of water (with 1mg/ml of Acian blue dye) and 2ml cyclohexane contaning 1,2-0-dodecylidene-xylose at a concentration of 0.1% and then mixed by vortex for 30s.

### Emulsion stability measurements

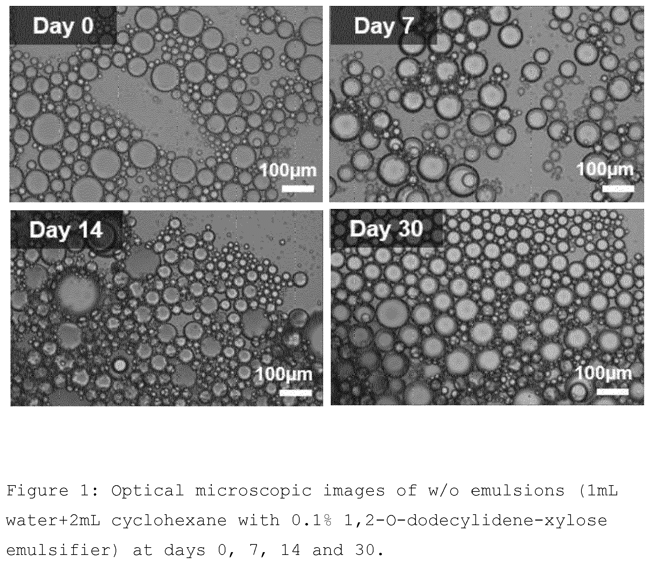

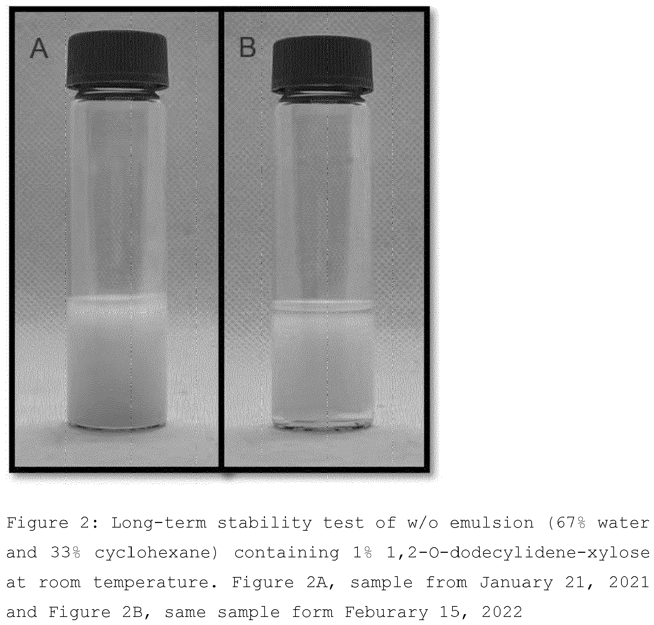

Emulsions were charcterized using a bright-field microscope (Leitz Ergolux) during the storage after preparation. Figure 1 shows that the aqueous bubbles in oil phase can maintain stable for at least 30 days without obvious coalescence. Besides, a water/oil emulsion (67% water and 33% cyclohexane) containing 1% 1,2-O-dodecylidene-xylose was kept for about 1 year to observe its possible destablization. Figure 2 shows a thin oil layer and a clarification layer was developed after 1 year, but most of the volume maintained the form of emulsion.

### Synthesis of 1,2-O-dodecylidene-xylose (6) and 3,5-O-dodecylidene-xylose (7) from xylose

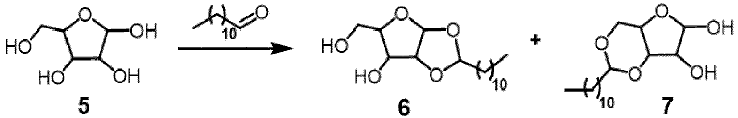

In a 1-neck round bottom flask, 1 molar equivalent of D-xylose was mixed with 0.9 molar equivalent dodecanal and 0.1 molar equivalents of sulfuric acid catalyst in dioxane. The reaction was conducted at 65°C for 24h. Then, the solution was neutralized with 1M NaOH solution until the pH value becomes around 7. The solution was concentrated on a rotavap with a bath temperature of 45 °C under reduced pressure (80 mbar). Then the residue viscous yellow oil was washed with brine solution and extracted with EtOAc. The organic phase was then evaporated on a rotavap and purified using column chromatography to obtain light yellow solid and yellowish oil. They were characterized by Heteronuclear Single Quantum Coherence Spectroscopy (HSQC) NMR.

### Synthesis of didodecylidene-xylose (8) from xylose

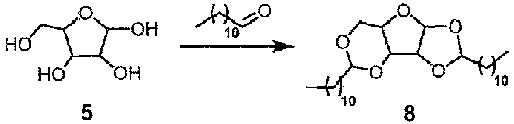

In a 1-neck round bottom flask, 1 molar equivalent of D-xylose was mixed with 2.05 molar equivalent dodecanal and 0.2 molar equivalents of sulfuric acid catalyst in dioxane. The reaction was conducted at 65°C for 24h. Then, the solution was neutralized with 1M NaOH solution until the pH value becomes around 7. The solution was concentrated on a rotavap with a bath temperature of 45 °C under reduced pressure (80 mbar). Then the resultant viscous pale-yellow oil was washed with brine solution and extracted with EtOAc. The organic phase was then evaporated on a rotavap and purified using flash chromatography. The product was collected and crystalized in hexane in the fridge to afford white didodecylidene-xylose crystals (8).

### Analytical methods

### Terminoloy

In the context of the present invention the expression CnALDxyl and 2CnALDxyl refer to xylose compounds, wherein the term "n" definies the length of the variable linear alkyl group. For example the term C12ALDxyl refers to 1,2-0-dodecylidene-xylose, the term C10ALDxyl refers to 1,2-O-decylidene-xylose and the term C8ALDxyl refers to 1,2-O-octylidene-xylose. On the other hand the expression 2CnALDxyl refers to similar xylose targets, where the term "n" defines the length of both variabel linear alkyl groups. For example the term 2C12ALDxyl refers to didodecylidene-xylose, the term 2C10ALDxyl refers to didecylidene-xylose and the term 2C8ALDxyl refers to dioctylidene-xylose.

### NMR

All NMR spectra (¹H, ¹³C, HSQC) were acquired using a Bruker Avance III 400 MHz spectrometer using the standard pulse sequences from Bruker.

### GC-MS

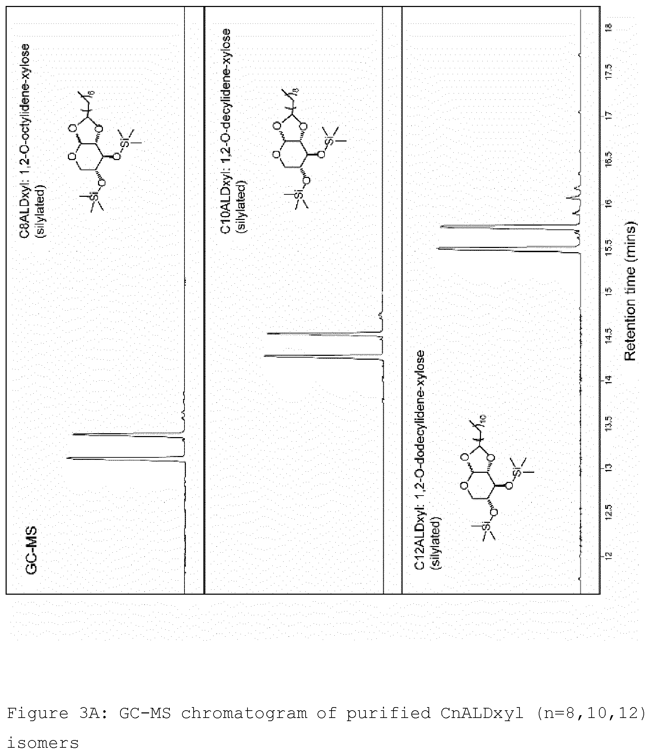

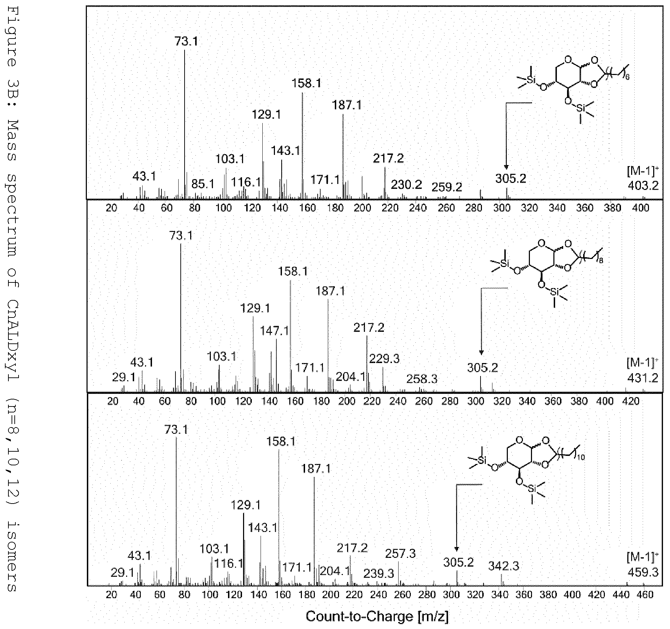

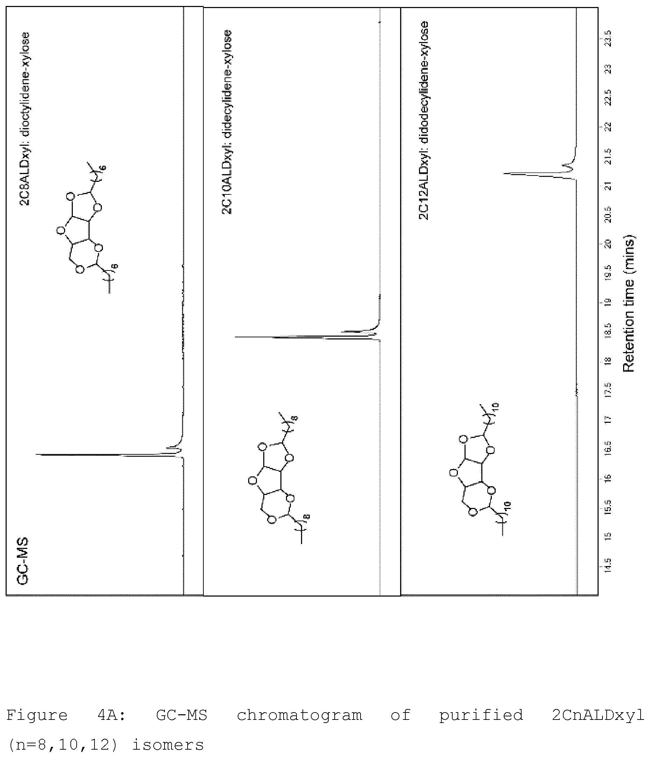

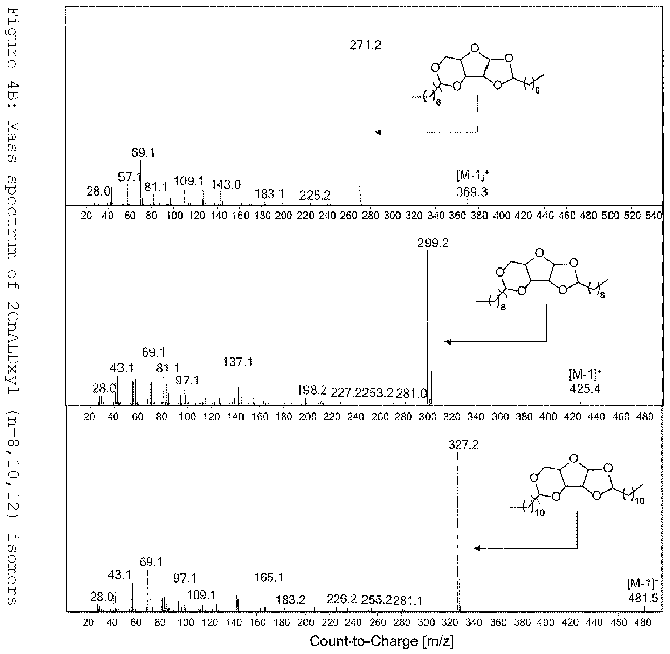

Gas chromatography-mass spectrometry spectra of the 2CnALDxyl (n=8,10,12; Figure 3A) and CnALDxyl (n=8,10,12; Figure 4A) isomers were obtained using an Agilent 7890B series GC equipped with a HP5-MS capillary column and an Agilent 5977A series Mass Spectroscopy detector (Figure 3B and 4B). A silylation derivatization was applied to CnALDxyl (n=8,10,12) by adding 100µL N-Methyl-N-(trimethylsilyl)-trifluoroacetamide (MSTFA) and 100µL pyridine and kept under r.t. for 30min before detection. The GC-MS method was performed as follows: The injection temperature was 300 °C. 1 µL of sample was injected with an autosampler in split mode (split ratio: 25:1). The column was initially kept at 40 °C for 3 min, then was heated at a rate of 30 °C min⁻¹ to 100 °C, followed by a heating rate of 40 °C min⁻¹ to 300 °C and held for 5 min.

### HPLC (pH2 aqueous-phase chromatography)

HPLC analyses for the accelerated aqueous decomposition of CnALDxyl were performed using a HPX-87H Column (300 mm x 7.8 mm; column temperature = 60°C) using pH2 water as eluent (flow rate = 0.6 ml•min⁻¹, Vᵢₙⱼ=20µL) with 1260 Refractive Index Detector (RID) (G1362A)

### Characterization data of xylose acetals

### 3-O-dodecyl-1,2-O-dodecylidene-xylose (Ie)

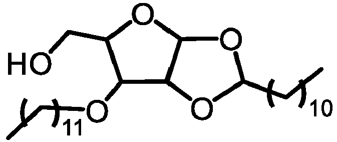

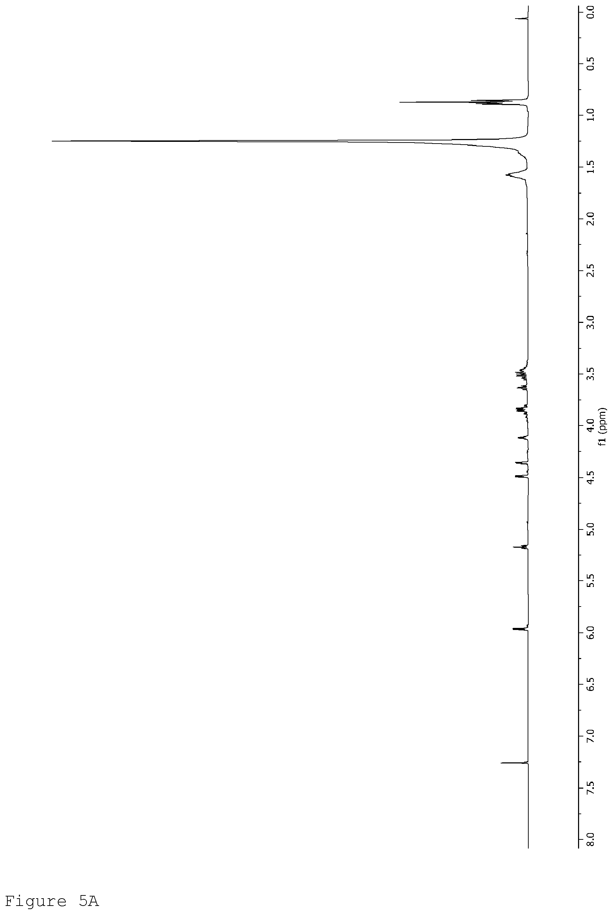

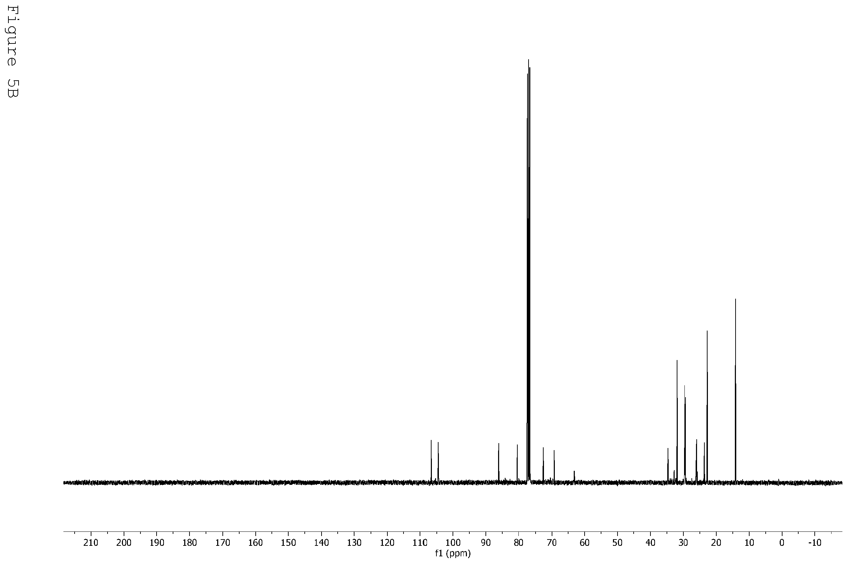

¹H NMR (400 MHz, CDCl₃) δ 5.96 (d, J = 3.7 Hz, 1H), δ 5.17 (t, *J* = 4.7 Hz, 1H), δ 4.49 (dd, *J* = 3.7 Hz, 1H), δ 4.34-4.37 (m, 1H), 4.09 - 4.14 (m, 1H), 3.78-3.89 (m, 2H), 3.40 - 3.56 (m, 2H), 1.20-1.36 (m, 36H), 0.87 (t, J = 7.08Hz, 6H). Figure 5A. ¹³C NMR (101 MHz, CDCl₃) δ 106.79, 104.68, 86.28, 80.59, 76.99, 72.72, 69.45, 34.77, 32.94, 32.05, 29.83, 29.80, 29.78, 29.76, 29.73, 29.67, 29.65, 29.58, 29.56, 29.53, 26.25, 23.70, 22.83, 14.25. Figure 5B

HRMS (ESI/QTOF) m/z: [M + Na]⁺ Calcd for C₂₉H₅₆NaO₅⁺ 507.4020; Found 507.4026.

### 5-O-dodecyl-1,2-O-dodecylidene-xylose (Id)

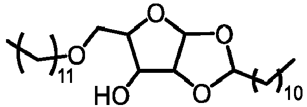

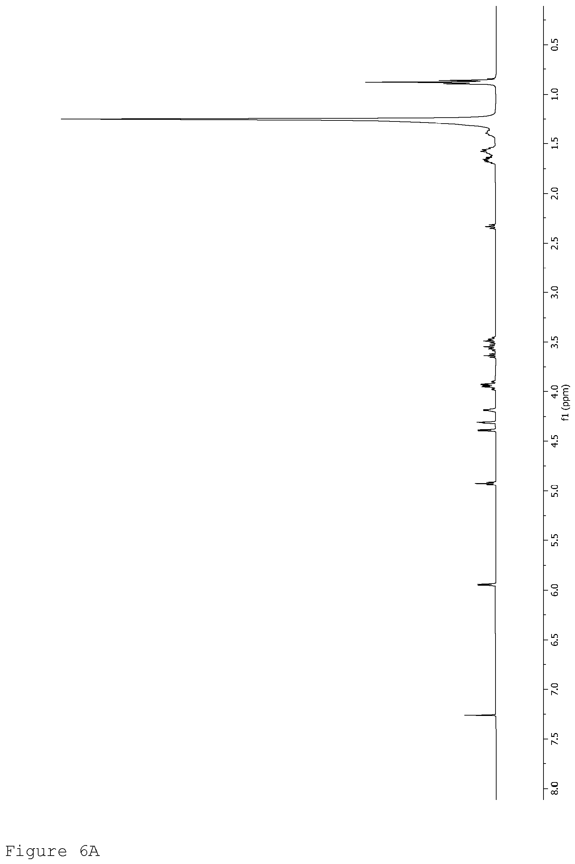

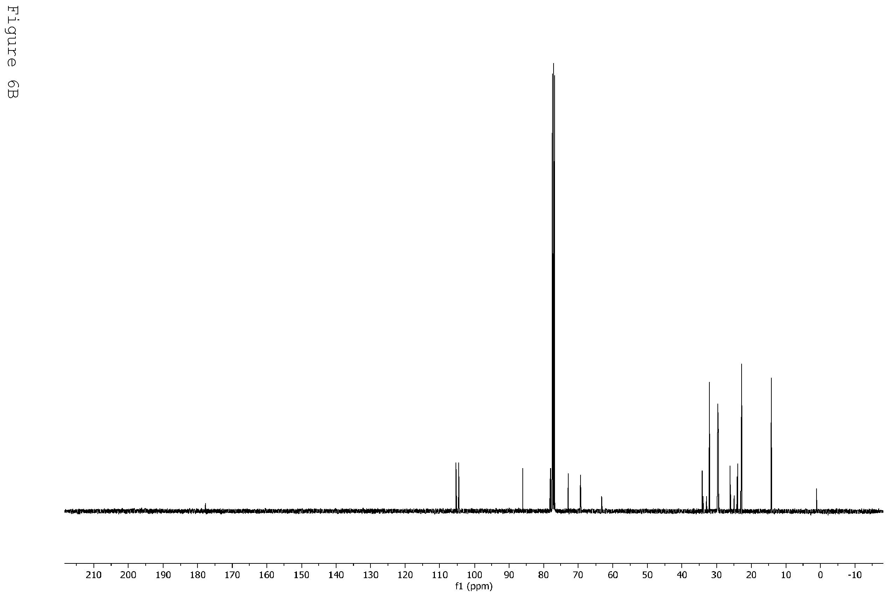

(b) ¹H NMR (400 MHz, CDCl₃) δ 5.94 (d, J = 4.0 Hz, 1H), 4.92 (t, J = 4.8 Hz, 1H), 4.39 (d, J = 4.0 Hz, 1H), 4.31 (d, J = 2.6 Hz, 1H), 4.18 (q, J = 3.1 Hz, 1H), 4.00 - 3.87 (m, 2H), 3.68 - 3.43 (m, 2H), 1.72 - 1.57 (m, 2H), 1.51-1.61 (m, 2H), 1.33 - 1.23 (m, 36H), 0.87 (t, *J* = 6.7 Hz, 6H). Figure 6A 13C NMR (101 MHz, CDCl₃) δ 105.40, 104.58, 86.02, 77.97, 76.88, 72.85, 69.29, 34.12, 32.94, 32.06, 29.81, 29.79, 29.77, 29.75, 29.73, 29.67, 29.64, 29.58, 29.55, 29.53, 26.09, 23.90, 22.83, 14.26. Figure 6B

HRMS (ESI/QTOF) m/z: [M + Ag]⁺ Calcd for C₂₉H₅₆AgO₅⁺ 591.3173; Found 591.3180.

### 1,2-O-dodecylidene-xylose (Ic)

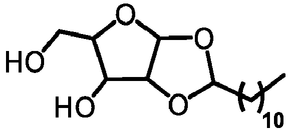

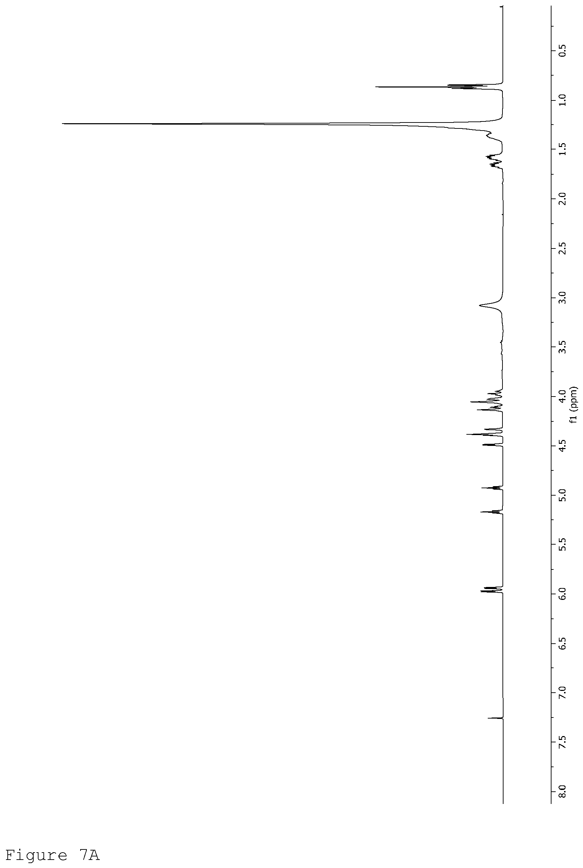

¹H NMR (400 MHz, CDCl₃) δ 5.96 (d, J = 3.7 Hz, 1H), 5.17 (t, J = 4.7 Hz, 0.52H), 4.92 (t, J = 4.8 Hz, 0.48H), 4.49 (d, J = 3.6 Hz, 1H), 4.42 - 4.31 (m, 2H), 4.16 - 3.93 (m, 4H), 1.16 - 1.42 (m, 18H), 0.86 (t, J = 6.7 Hz, 6H). Figure 7A

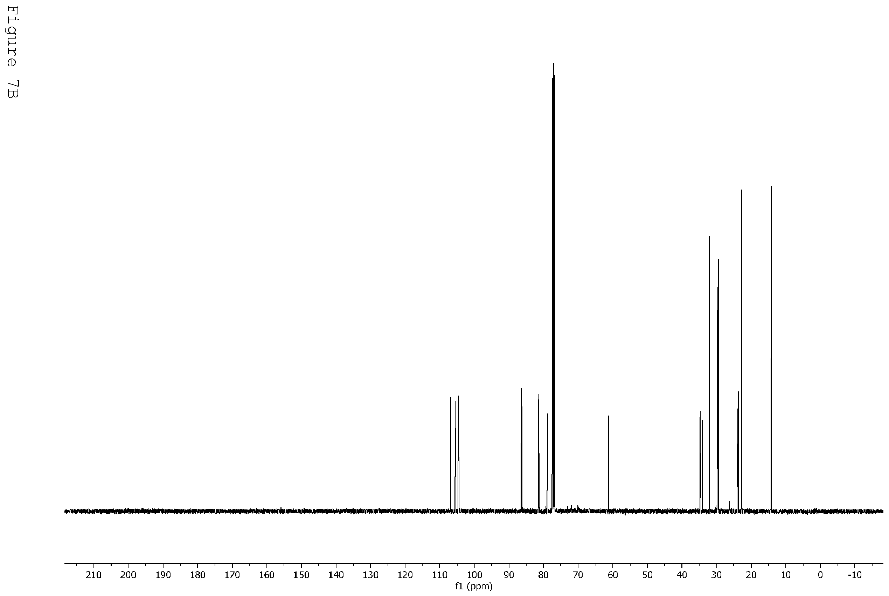

¹³C NMR (101 MHz, CDCl₃) δ 106.91, 105.62, 104.65, 104.55, 86.43, 86.22, 81.47, 78.79, 77.09, 76.96, 61.33, 61.20, 34.72, 34.09, 32.03, 29.80, 29.76, 29.75, 29.73, 29.71, 29.63, 29.62, 29.56, 29.54, 29.48, 29.46, 23.85, 23.65, 22.80, 14.24. Figure 7B

HRMS (nanochip-ESI/LTQ-Orbitrap) m/z: [M + H]⁺ Calcd for C₁₇H₃₃O₅⁺ 317.2323; Found 317.2318.

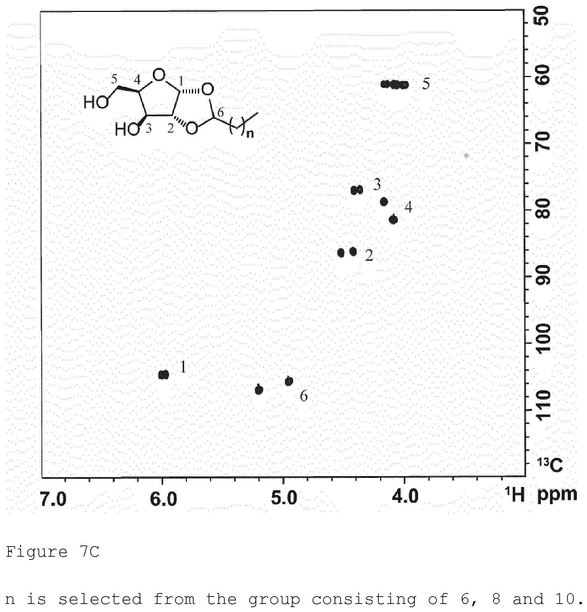

### Characterization of 1,2-O-dodecylidene-xylose via HSQC-NMR Figure 7C

### Characterization data of CnALDxyl and 2CnALDxyl C12ALDxyl (1,2-O-dodecylidene-xylose) (IVa)

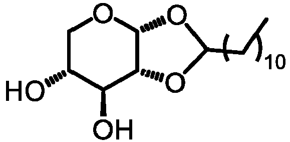

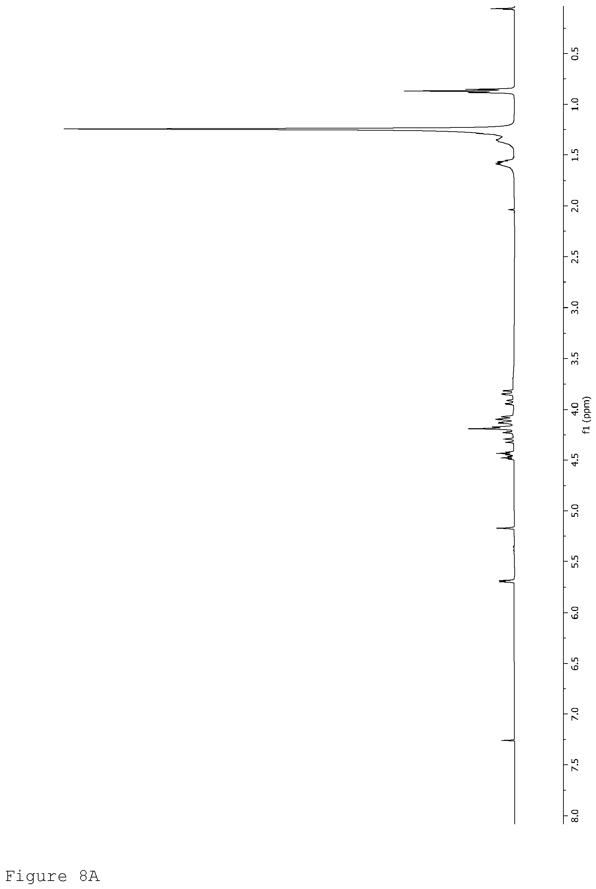

¹H NMR (400 MHz, CDCl₃) δ 5.69 (d, J = 3.8 Hz, 0.6H), 5.17 (s, 0.4H), 4.51-4.40 (m, 1H), 4.25 - 4.16 (m, 2H), 4.16 - 4.05 (m, 2H), 4.00 - 3.78 (m, 1H), 1.65-1.53 (m, 2H), 1.41 - 1.21 (m, 18H), 0.87 (t, J = 6.7 Hz, 3H). Figure 8A

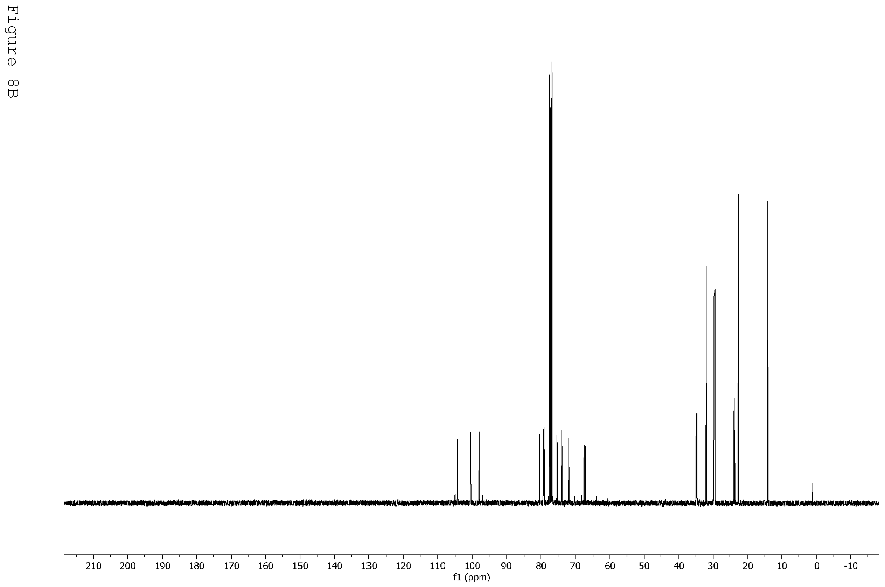

¹³C NMR (101 MHz, CDCl₃) δ 104.32, 100.52, 80.46, 73.94, 71.85, 67.40, 34.94, 34.83, 29.69, 29.64, 29.55, 29.47, 23.69, 22.80, 14.24. Figure 8B

### C10ALDxyl(1,2-O-decylidene-xylose) (IVb)

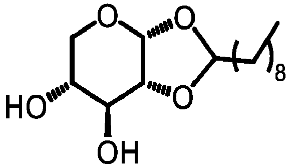

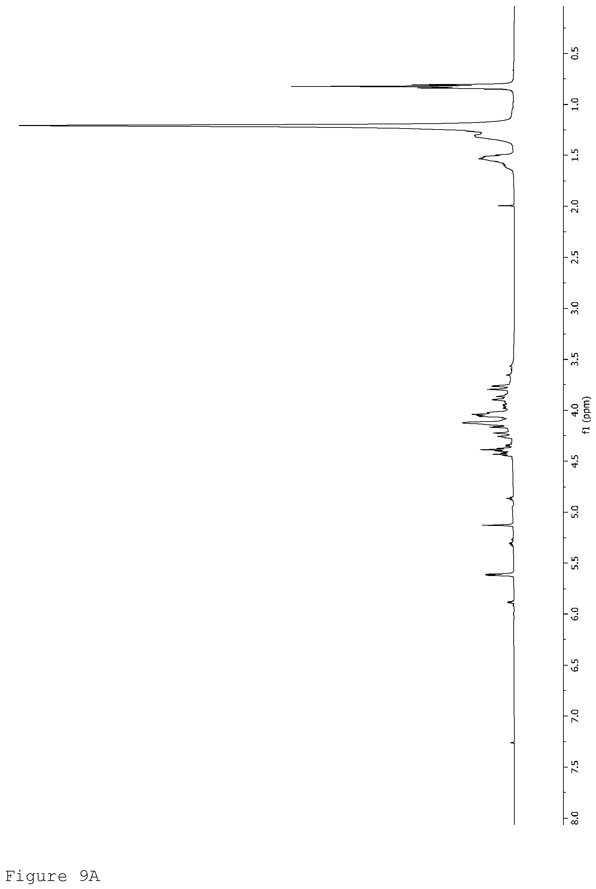

¹H NMR (400 MHz, CDCl₃) δ 5.61 (d, J = 3.7 Hz, 0.6H), 5.13 (s, 0.4H), 4.46-4.30 (m, 1H), 4.30 - 4.12 (m, 1H), 4.15 - 4.09 (m, 1H), 4.09 - 4.02 (m, 1H), 4.04 - 3.82 (m, 1H), 3.82-3.73 (m, 1H), 1.59 - 1.48 (m, 2H), 1.36 - 1.10 (m, 14H), 0.82 (t, J = 6.7 Hz, 3H). Figure 9A

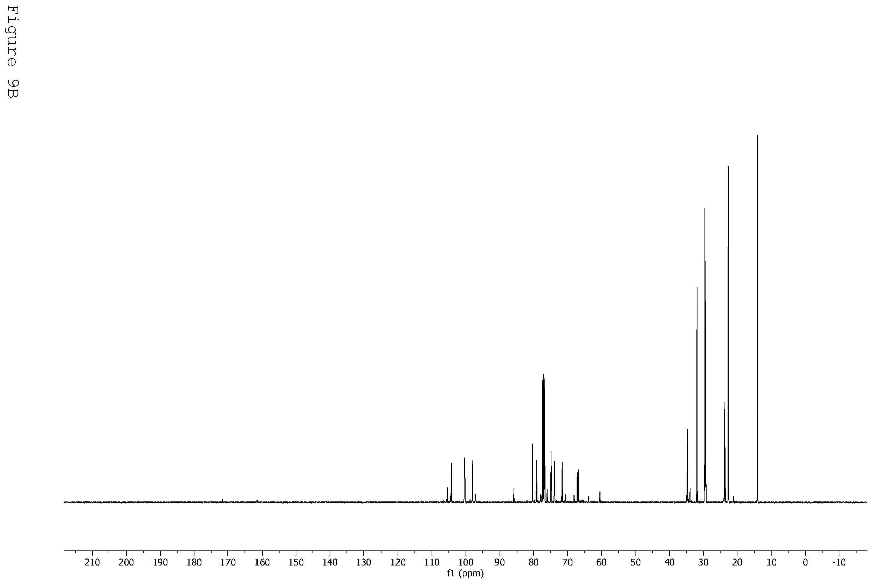

¹³C NMR (101 MHz, CDCl₃) δ 104.16, 100.42, 80.32, 74.93, 71.58, 67.20, 34.47, 33.96, 29.51, 29.42, 29.39, 29.31, 23.29, 22.80, 14.09. Figure 9B

### C8ALDxyl(1,2-O-octylidene-xylose) (IVc)

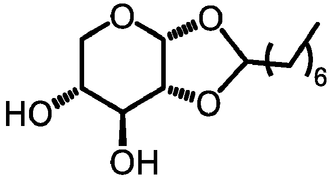

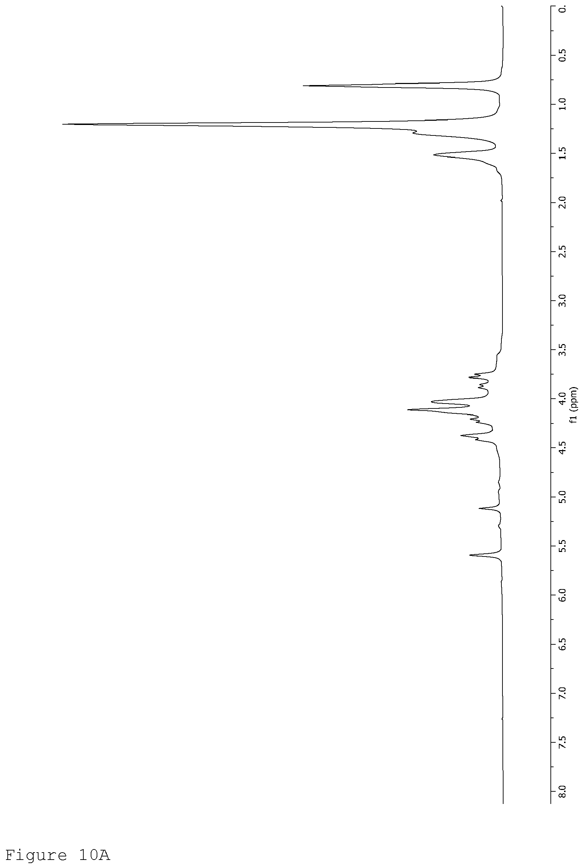

¹H NMR (400 MHz, CDCl₃) δ 5.59 (s, 0.66H), 5.12 (s, 0.34H), 4.44-4.34 (m, 1H), 4.26-4.18 (m, 1H), 4.16-4.06 (m, 3H), 4.07 - 3.99 (m, 1H), 1.72 - 1.43 (m, 2H), 1.40-0.95 (m, 10H), 0.82 (t, *J* = 6.1 Hz, 3H). Figure 10A

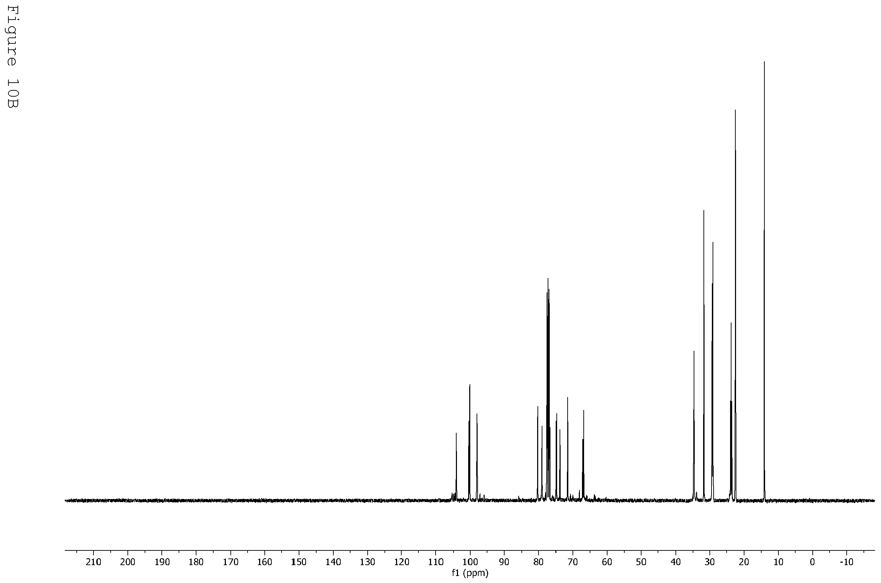

¹³C NMR (101 MHz, CDCl₃) δ 104.11, 100.37, 80.27, 73.81, 71.52, 67.14, 34.65, 31.71, 29.31, 29.28, 29.12, 29.10, 23.58, 22.57, 14.02. Figure 10B

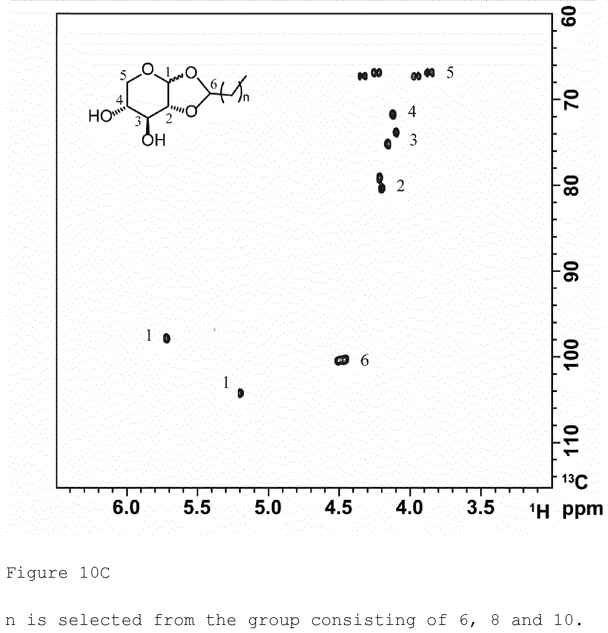

### Characterization of 1,2-O-octylidene-xylose via HSQC-NMR, Figure 10C

### 2C12ALDxyl(didodecylidene-xylose) (Ia)

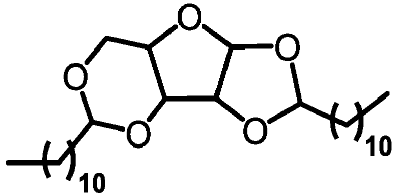

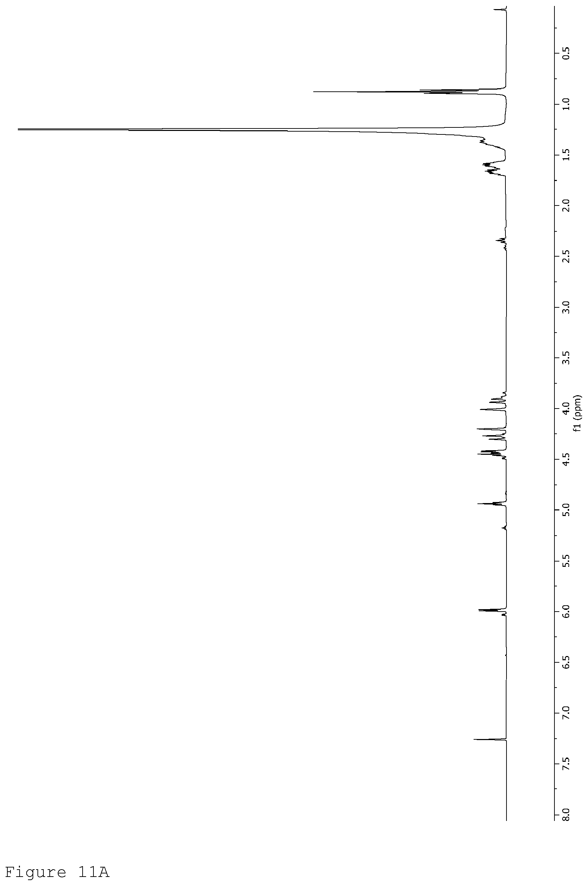

¹H NMR (400 MHz, CDCl₃) δ 6.01 (d, J = 3.8 Hz, 1H), 4.94 (t, J = 4.8 Hz, 1H), 4.51 - 4.39 (m, 2H), 4.32-4.22 (m, 1H), 4.20 (d, J = 2.1 Hz, 1H), 4.02-3.98 (m, 1H), 3.96 - 3.88 (m, 1H), 1.72 - 1.55 (m, 4H), 1.43 - 1.17 (m, 36H), 0.87 (t, *J* = 6.98 Hz, 6H). Figure 11A

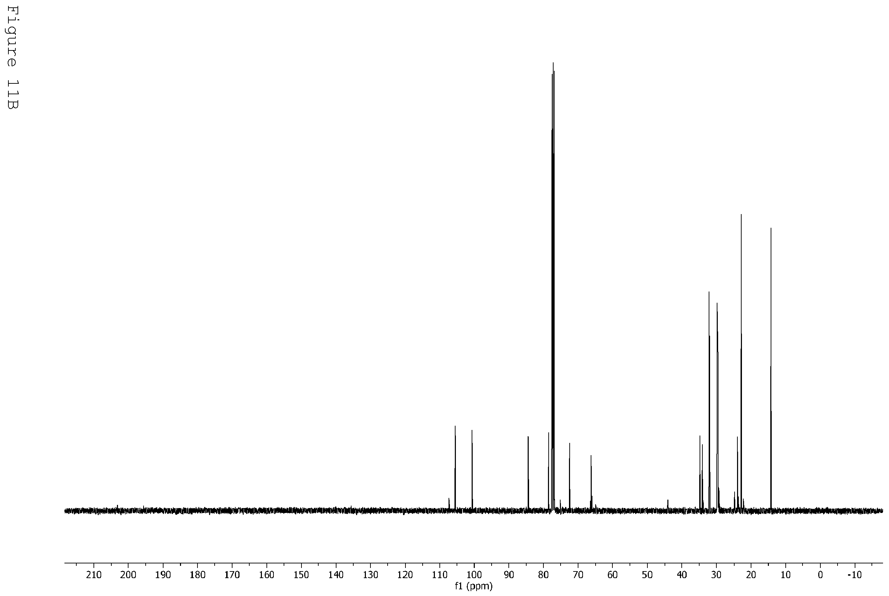

¹³C NMR (101 MHz, CDCl₃) δ 105.48, 105.34, 100.57, 84.42, 78.48, 72.55, 66.22, 34.81, 34.77, 32.06, 32.05, 29.76, 29.68, 29.64, 29.62, 29.58, 29.51, 29.48, 29.39, 29.32, 29.21, 24.85, 23.97, 23.92, 23.80, 22.83, 14.26. Figure 11B

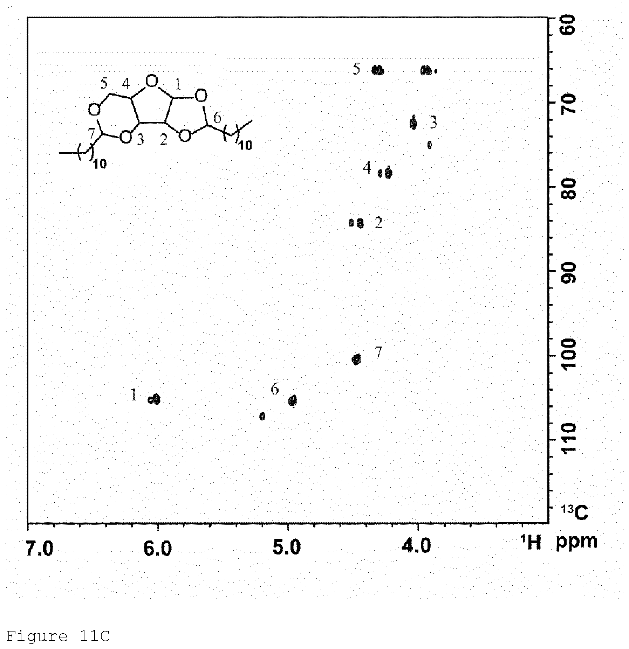

### Characterization of didodecylidene-xylose via HSQC-NMR, Figure 11C

### 2C10ALDxyl(didecylidene-xylose) (Ia)

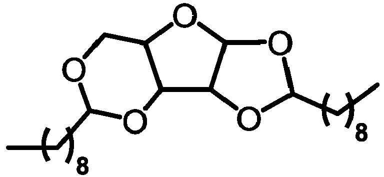

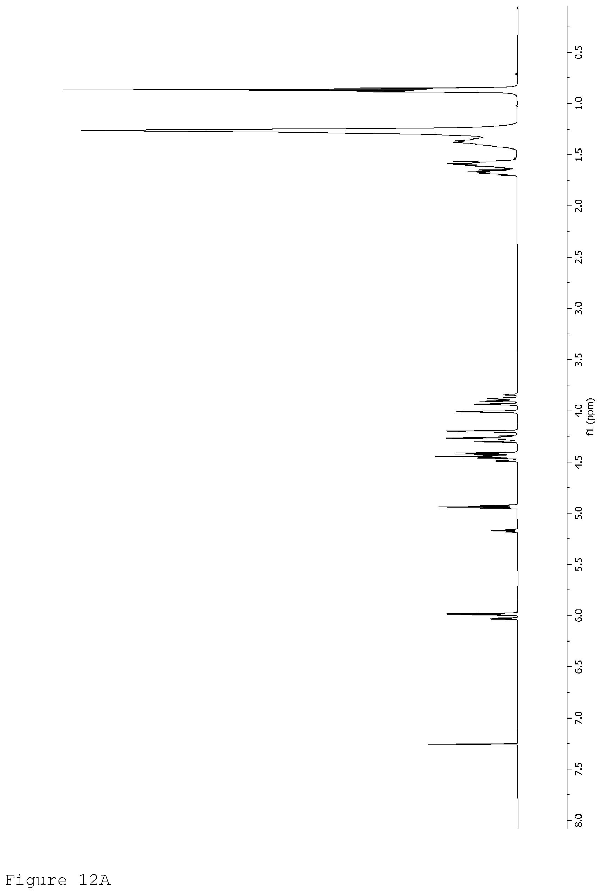

¹H NMR (400 MHz, CDCl₃) δ 5.99 (d, *J* = 4.0 Hz, 1H), 4.94 (t, J = 4.8 Hz, 1H), 4.50 - 4.39 (m, 1H), 4.33 - 4.23 (m, 2H), 4.20 (d, *J* = 2.2 Hz, 1H), 3.96 - 3.82 (m, 2H), 1.72 - 1.52 (m, 4H), 1.44 - 1.22 (m, 28H), 0.87 (t, *J* = 6.8 Hz, 6H). Figure 12A

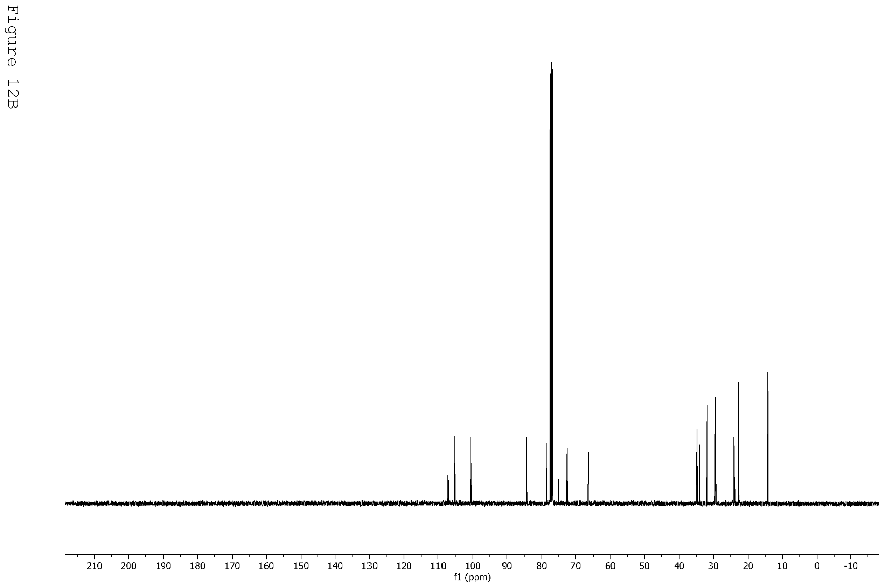

¹³C NMR (101 MHz, CDCl₃) δ 107.28, 105.45, 100.57, 84.41, 78.49, 75.09, 72.55, 66.22, 34.77, 34.07, 32.02, 32.00, 29.63, 29.62, 29.60, 29.58, 29.55, 29.50, 29.43, 29.42, 23.96, 23.92, 23.80, 23.66, 22.81, 14.25. Figure 12B

### 2C8ALDxyl(dioctylidene-xylose) (Ia)

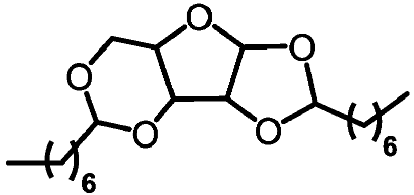

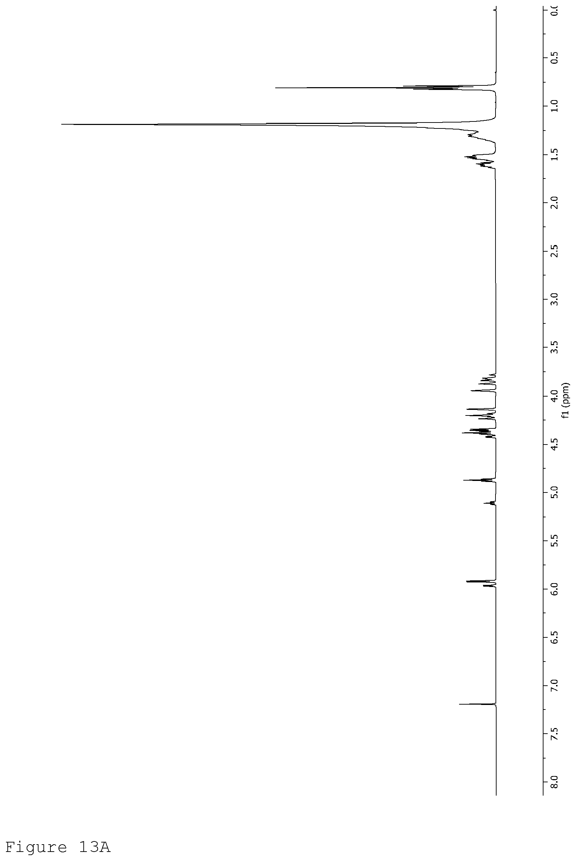

¹H NMR (400 MHz, CDCl₃) δ 5.99 (d, *J* = 4.0 Hz, 1H), 4.94 (t, J = 4.8 Hz, 1H), 4.52 - 4.39 (m, 2H), 4.33 - 4.23 (m, 1H), 4.20 (d, J = 2.2 Hz, 1H), 4.04-3.98 (m, 1H), 3.96 - 3.82 (m, 1H), 1.72 - 1.52 (m, 4H), 1.45 - 1.14 (m, 20H), 0.90 - 0.82 (m, 6H). Figure 13A

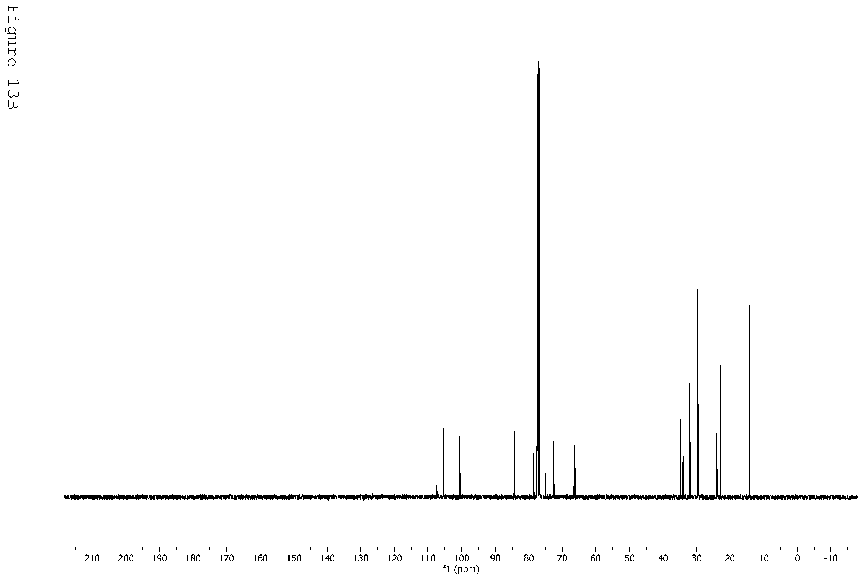

¹³C NMR (101 MHz, CDCl₃) δ 107.28, 105.48, 100.57, 84.41, 78.49, 75.09, 72.55, 66.40, 34.77, 34.07, 31.87, 31.85, 29.54, 29.51, 29.46, 29.29, 29.28, 23.96, 23.92, 23.80, 23.66, 22.77, 22.75, 14.22. Figure 13B

### 2-((dodecyloxy)methyl)tetrahydrofuran-3,4-diol (Va)

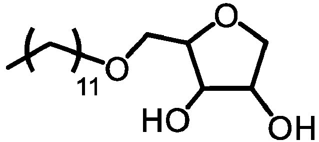

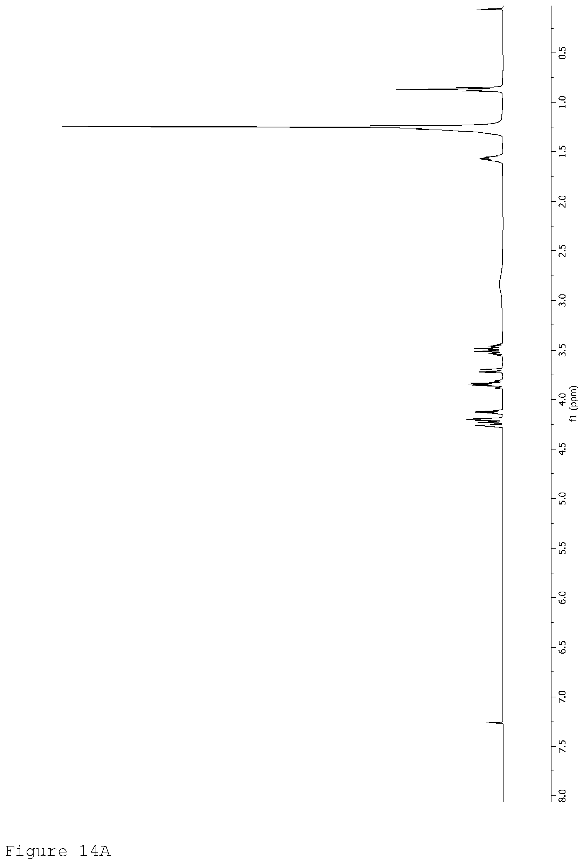

¹H NMR (400 MHz, CDCl₃) δ 4.27 (dt, J = 3.9, 1.6 Hz, 1H), 4.24 - 4.17 (m, 2H), 4.16 - 4.09 (m, 1H), 3.91 - 3.78 (m, 2H), 3.74 - 3.67 (m, 1H), 3.58 - 3.42 (m, 2H), 1.63 - 1.53 (m, 2H), 1.34 - 1.22 (m, 18H), 10.87 (t, J = 6.8 Hz, 3H). Figure 14A

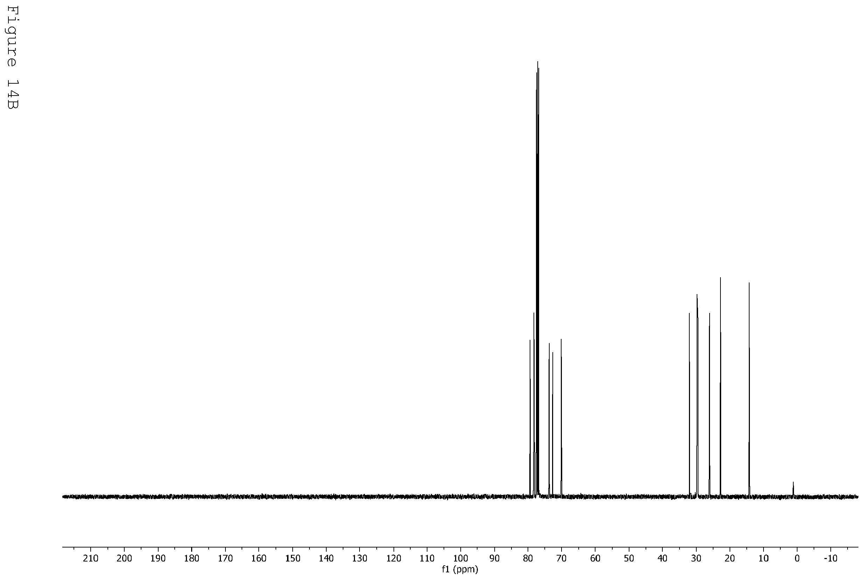

¹³C NMR (101 MHz, CDCl₃) δ 79.39, 78.24, 78.16, 73.70, 72.62, 70.06, 32.04, 31.56, 29.78, 29.75, 29.72, 29.67, 29.61, 29.53, 26.13, 22.81, 14.24. Figure 14B

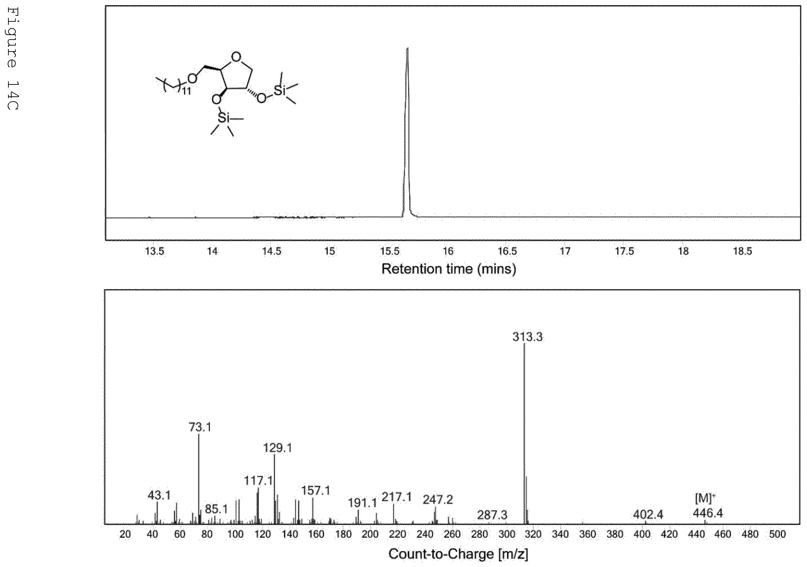

HRMS (ESI/QTOF) m/z: [M + Na]⁺ Calcd for C₁₇H₃₄NaO₄⁺ 325.23452; Found 325.23455. Figure 14C

### Interfacial tension measurements

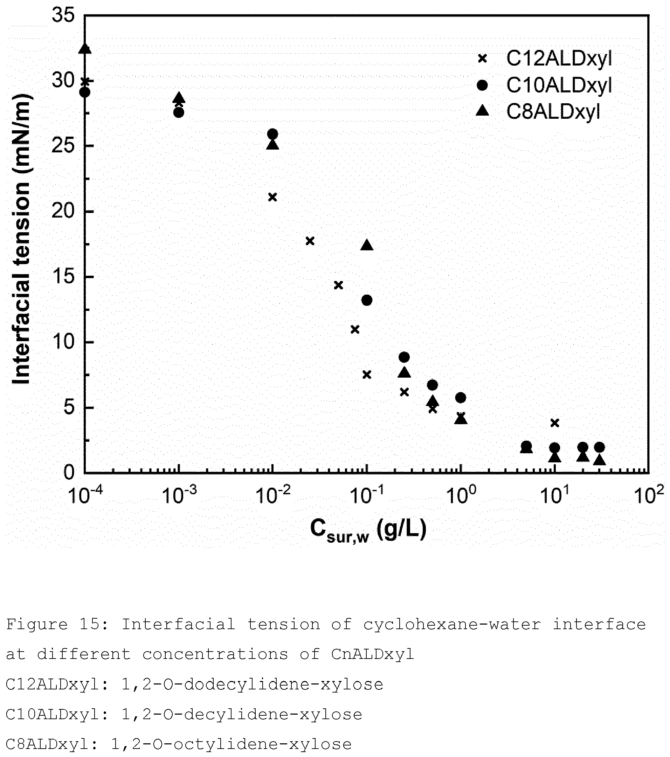

To test the amphiphilic properties of each molecule, we measured the water/oil interfacial tension using pendant drop test. In a typical test, we inject the organic solution with surfactants slowly into the water phase with a bent needle with 1mm diameter. The interfacial tension is calculated by the force balance with the buoyancy force. The critical micelle concentration (CMC) can be obtained by checking the critical point of interfacial tension-concentration graph. Figure 15 shows that C12ALDxyl has lowest CMC (0.35mg/mL) among tail length between 8-12. The CMC of C10AcXyl and C8AcXyl are around 2.5mg/mL.

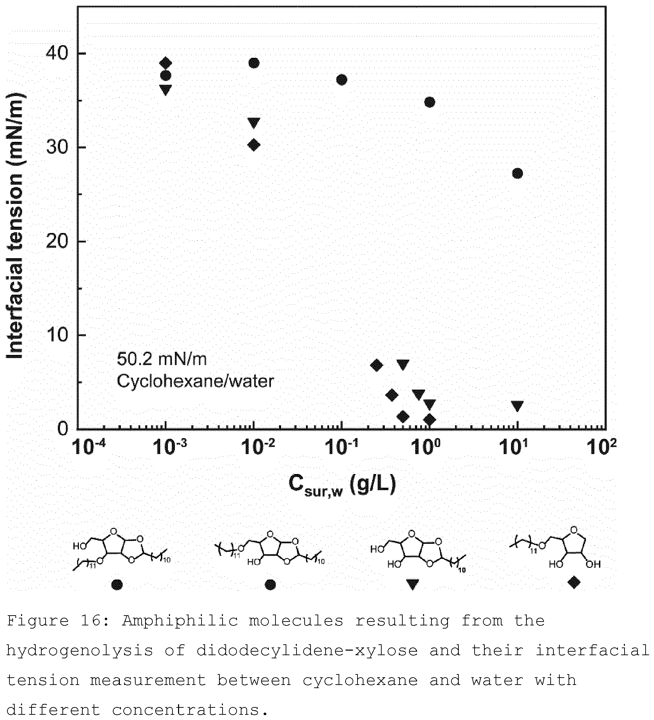

Figure 16 shows that the CMC of 2-((dodecyloxy)methyl) tetrahydrofuran-3,4-diol (e) is around 0.5g/L, and it can reduce the interfacial tension (cyclohexane/water) to a plateau value about 1.0 mN/m. 1,2-O-dodecylidene-xylose (d) has a CMC around 1g/L and induce a decrease of the interfacial tension (cyclohexane/water) to a plateau value about 2.7 mN/m.

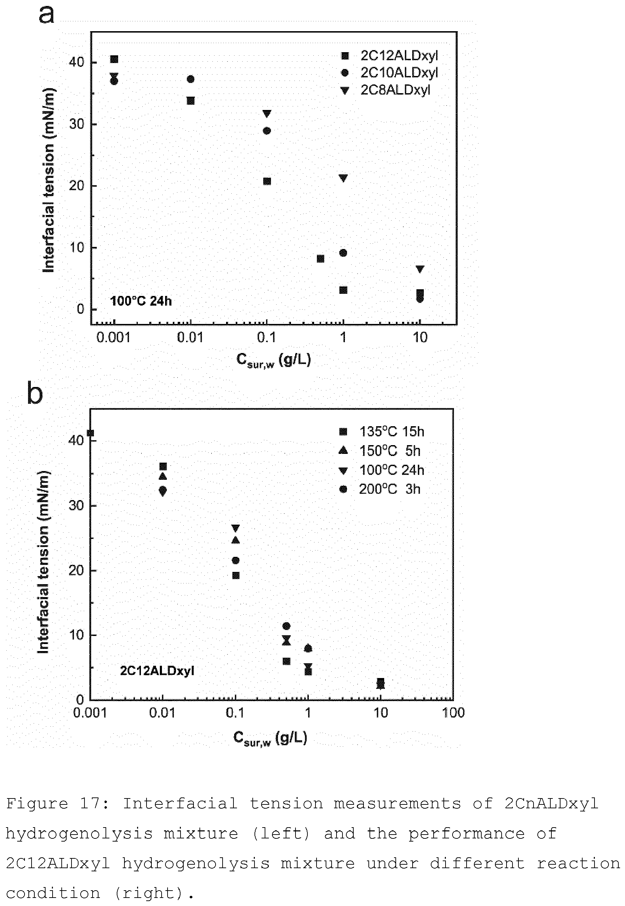

The reaction mixture without purification also has amphiphilic properties, which vary depending on the alkyl chain length shown by Figure 17a. Among the length from C₈ to C₁₂, 2C12ALDxyl reaction mixture demonstrates the best ability of reducing the interfacial tension to a plateau value about 3 mN/m (cyclohexane/water). And the amphiplic properties of reaction mixture show differences under different hydrogenolysis conditions. For example, 135 °C for 15h lowered the interfacial tension(cyclohexane/water) to 6 mN/m at 0.5g/L, while 200 °C for 3h lowered the interfacial tension(cyclohexane/water) to 11.5 mN/m at 0.5g/L shown in Figure 17b.

### Accelerated aqueous decomposition test

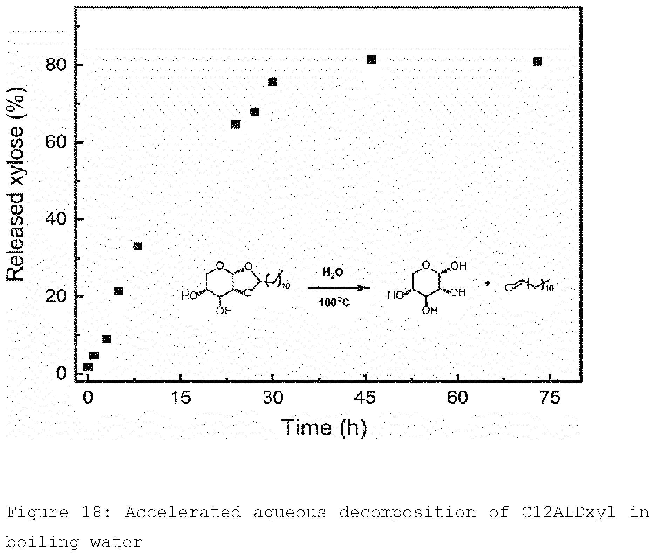

To gain insight into the degradation products of C12ALDxyl in water, an accelerating aging test was performed by boiling it in water. Samples were taken at various time points and analyzed by HPLC (pH2 aqueous-phase chrmatography, HPX-87H Column (300 mm x 7.8 mm; 125-0140), 1260 Refractive Index Detector (RID) (G1362A), flow rate = 0.6 ml•min⁻¹, Vᵢₙⱼ=20µL, column temperature = 60 °C). It shows that C12ALDxyl can be cleaved into xylose and fatty aldehyde in boiling water in 2 days. And to our knowledge, fatty aldehydes can be oxidized into fatty acids catalyzed by the aldehyde dehydrogenase enzyme. Xylose and fatty acids are readily biodegradable. The result shows that the C12ALDxyl can be easily decomposed and degraded after use (Figure 18).

## Claims

1. Compound of the general formula (I), (II) and (III) 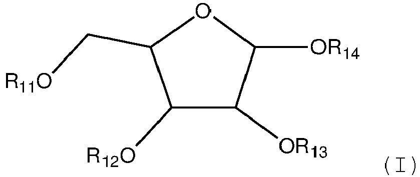 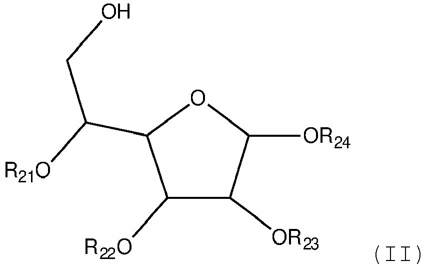 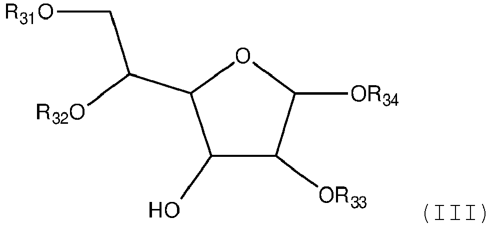
wherein R₁₁ and R₁₂ or R₂₁ and R₂₂ or R₃₁ and R₃₂ are both hydrogen or form together with CHR₅₀ a cyclic moiety or one of R₁₁ and R₁₂ or R₂₁ and R₂₂ or R₃₁ and R₃₂ is hydrogen, and the other is -CH₂R₇₀ and
wherein R₁₃ and R₁₄ or R₂₃ and R₂₄ or R₃₃ and R₃₄ are both hydrogen or form together with CHR₆₀ a cyclic moiety,
R₅₀ and R₆₀ are different form each other and are selected from the group consisting of -R₇₀, -ZR₇₀, -Z-OH, -Z-NH₂, - Z-SH, -Z-OC(O)R₇₀, -OC(O)R₇₀, -COOH, -C(O)NH₂, -C(O)NH-R₇₀, -C(O)N-(R₇₀)₂, -COOR₇₀, -Z-COOH, -Z-C(O)NH-R₇₀, -Z-C(O)NH₂, -Z-C(O)N-(R₇₀)₂, -Z-COOR₇₀, -CH(COOH)₂, -CH(COOR₇₀)₂, and - Z-SO₃⁻
wherein R₇₀ is selected from the group consisting of a linear or branched C₁ to C₂₀ alkyl, (C₁ to C₁₀)-alkyloxy-(C₁ to C₁₀)-alkyl, C₂ to C₁₀ alkenyl, C₆ to C₁₂ aryl, C₃ to C₁₀ cycloalkyl, cycloalkylalkyl and cycloalkylalkenyl,
wherein Z is a linear or branched C₁ to C₁₀ alkyl, linear or branched C₃ to C₁₀ cycloalkyl, a linear or branched C₆ to C₁₀ aryl or a (C₁ to C₁₀)-alkyloxy- (C₁ to C₁₀)-alkyl, cycloalkylalkyl and cycloalkylalkenyl,
with the proviso that
not all of R₁₁, R₁₂, R₁₃ and R₁₄ or R₂₁, R₂₂, R₂₃ and R₂₄ or R₃₁, R₃₂, R₃₃ and R₃₄ are hydrogen and
if one of R₁₁ and R₁₂ or R₂₁ and R₂₂ or R₃₁ and R₃₂ is hydrogen and the other is -CH₂R₇₀, R₁₃ and R₁₄ or R₂₃ and R₂₄ or R₃₃ and R₃₄ form together with -CHR₆₀ a cyclic moiety, wherein R₆₀ is R₇₀.

2. Compound according to claim 1, wherein the compound of formula (I), (II) or (III) is selected from the group consisting of

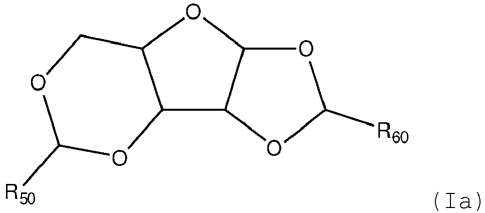

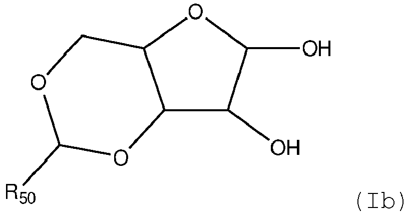

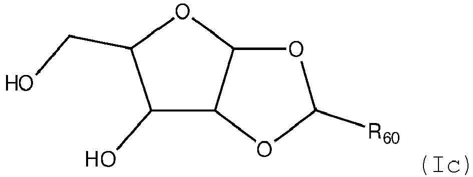

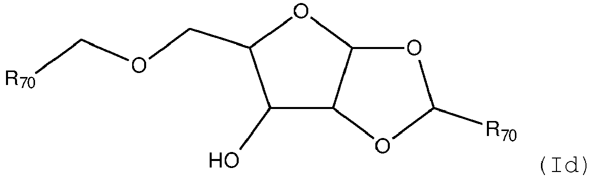

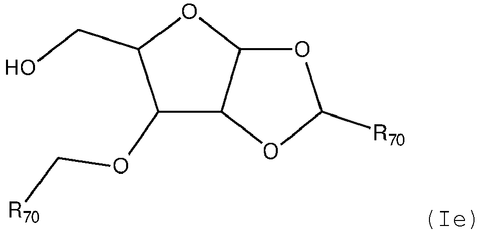

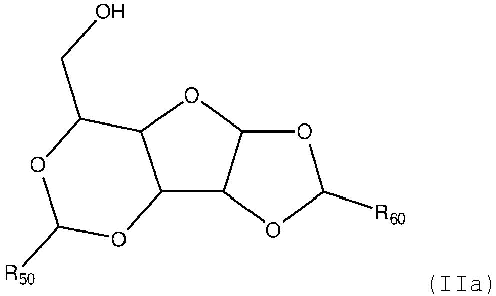

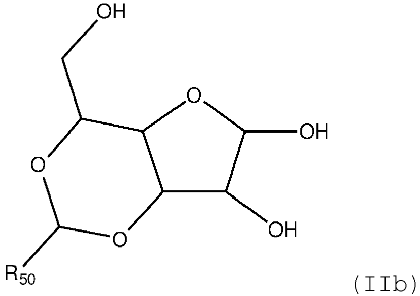

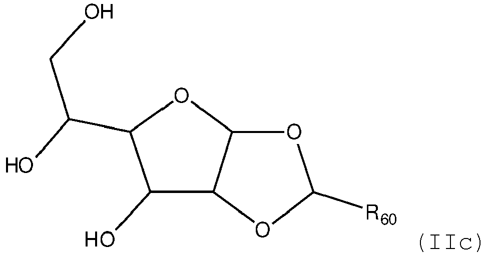

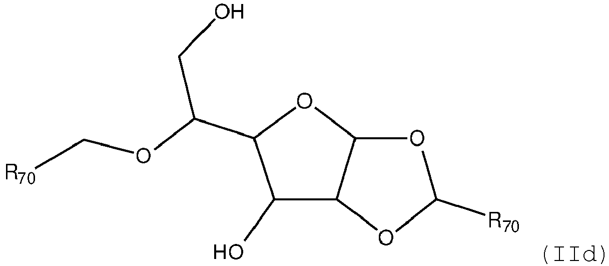

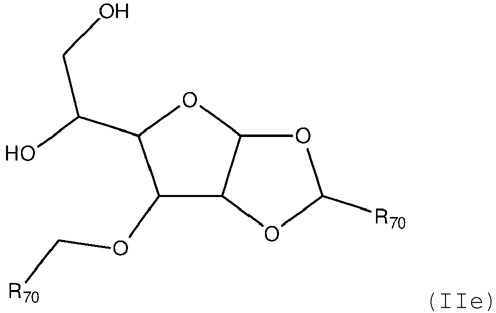

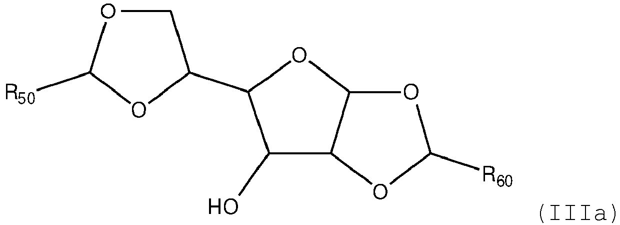

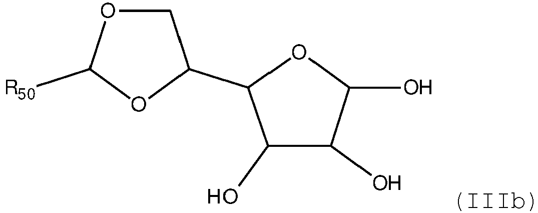

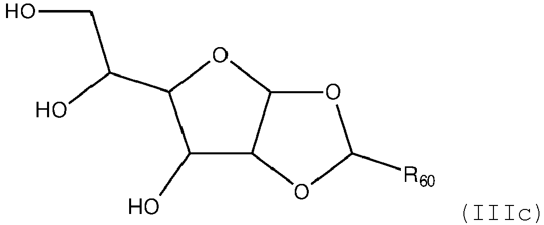

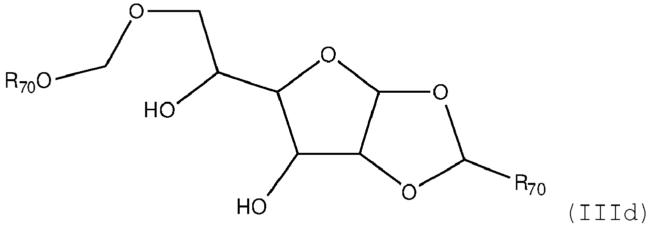

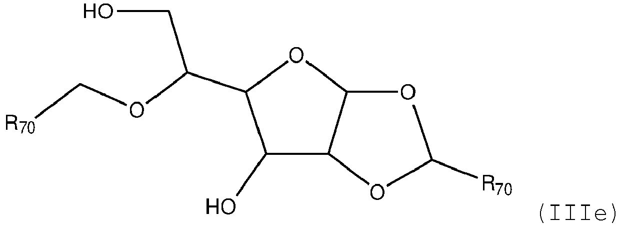

3. Compound of the general formula (I), (II) and (III) according to Claims 1 or 2, wherein
one of R₅₀ and R₆₀ is selected from the group consisting of -Z-OC(O)R₇₀, -COOR₇₀ and -Z-COOR₇₀ and
and the other is selected from the group consisting of - Z-OH, -COOH and -Z-COOH.

4. Compound according to claim 3, wherein Z is a linear or branched C₁ to C₁₀ alkyl, preferably a linear C₁ to C₅ alkyl, most preferably a linear C₁ to C₃ alkyl.

5. Compound of the general formula (I), (II) and (III) according to Claims 1 or 2,
wherein R₁₁ and R₁₂ or R₂₁ and R₂₂ or R₃₁ and R₃₂ are both hydrogen or form together with CHR₅₀ a cyclic moiety, or wherein R₁₃ and R₁₄ or R₂₃ and R₂₄ or R₃₃ and R₃₄ are both hydrogen or form together with CHR₆₀ a cyclic moiety, and
R₅₀ or R₆₀ are a linear C₁ to C₁₀ alkyl.

6. Compound of the general formula (Ia) according to Claim 2,
wherein R₅₀ and R₆₀ are both a linear C₁ to C₂₀ alkyl, preferably a linear C₇ to C₁₁ alkyl and more preferably are selected from the group consisting of -(CH₂)₆CH₃, - (CH₂)₈CH₃ and - (CH₂)₁₀CH₃.

7. Compound of the general formula (Ic) according to Claim 2,
wherein R₆₀ is R₇₀ and R₇₀ is a linear C₁ to C₂₀ alkyl, preferably a linear C₇ to C₁₁ alkyl and more preferably a linear C₁₁ alkyl.

8. Compound of the general formula (Id) and (Ie) according to Claim 2,
wherein R₇₀ is a linear C₁ to C₂₀ alkyl, preferably a linear C₇ to C₁₁ alkyl and more preferably a linear C₁₁ alkyl.

9. Compound of the general formula (I), (II) and (III) according to Claims 1 or 2,
wherein R₁₁ and R₁₂ or R₂₁ and R₂₂ or R₃₁ and R₃₂ form together with CHR₅₀ a cyclic moiety and
wherein R₁₃ and R₁₄ or R₂₃ and R₂₄ or R₃₃ and R₃₄ form together with CHR₆₀ a cyclic moiety, and
R₅₀ and R₆₀ are different and are selected from the group consisting of -COOH and -C(O)NH-R₇₀, and
wherein -R₇₀ is preferably a linear or branched C₁ to C₂₀ alkyl, more preferably a linear C₅ to C₁₅ alkyl and most preferably a linear C₁₀ to C₁₅ alkyl.

10. Compound of the general formula (I), (II) and (III) according to Claim 7,
wherein R₅₀ is -C(O)NH-R₇₀ and R₆₀ is -COOH, preferably - R₇₀ is a linear or branched C₁ to C₂₀ alkyl, more preferably a linear C₅ to C₁₅ alkyl and most preferably a linear C₁₀ to C₁₅ alkyl.

11. Compound of the general formula (I), (II) and (III) according to Claims 1 or 2
wherein R₁₁ and R₁₂ or R₂₁ and R₂₂ or R₃₁ and R₃₂ form together with CHR₅₀ a cyclic moiety and
wherein R₁₃ and R₁₄ or R₂₃ and R₂₄ or R₃₃ and R₃₄ form together with CHR₆₀ a cyclic moiety, and
R₅₀ and R₆₀ are different and are selected from the group consisting of -Z-OH and -Z-SO₃⁻, preferably -Z is a linear or branched C₁ to C₁₀ alkyl, more preferably a linear C₁ to C₅ alkyl and most preferably a linear C₁ to C₃ alkyl.

12. Compound of the general formula (I), (II) and (III) according to Claim 9
wherein R₅₀ is -Z-OH and R₆₀ is -Z-SO₃⁻.

13. Use of the compounds according to one of the Claims 1 to 12 as surfactants, preferably as emulsifiers, foam stabilizers, wetting agents, emollients in cosmetic products, surfactants in food products, as anti-spattering agents for frying or as surfactant in pharmaceutical products.

14. Use of the compounds according Claims 9 or 10 as surfactants preferably in oil recovery, in assisted synthesis of nanoparticle, as foam boosters and additives in cleaning formulations for laundry applications.

15. Use of the compounds according to Claims 11 or 12 as foaming agents or detergents in shampoos, body washes and soaps.
